# EUROPEAN PATENT SPECIFICATION

(11) **EP 1 692 143 B1**
(45) Date of publication and mention of the grant of the patent: **12.11.2008**
(21) Application number: 04803724.6
(22) Date of filing: 09.12.2004
(51) Int. Cl.: C07D 513/06, A61K 31/554, A61P 25/28

(54) **TRICYCLIC INDOLE HYDROXYETHYLAMINE DERIVATIVES AND THEIR USE IN THE TREATMENT OF ALZHEIMER'S DISEASE**
TRICYCLISCHE INDOLHYDROXYETHYLAMINDERIVATE UND DEREN VERWENDUNG BEI DER BEHANDLUNG VON ALZHEIMER-KRANKHEIT
DERIVES D'INDOLE HYDROXYETHYLAMINE TRICYCLIQUES ET LEUR UTILISATION DANS LE TRAITEMENT DE LA MALADIE D'ALZHEIMER

(30) Priority: 12.12.2003 GB 0328900
(43) Date of publication of application: 23.08.2006
(73) Proprietor: GLAXO GROUP LIMITED, Greenford, Middlesex UB6 ONN (GB)
(72) Inventor: REDSHAW, Sally, GlaxoSmithKline, Harlow,Essex CM19 5AW (GB); DEMONT, Emmanuel Hubert, GlaxoSmithKline, Stevenage, Hertfordshire SG1 2NY (GB); WALTER, Daryl Simon, GlaxoSmithKline, Harlow, Essex CM19 5AW (GB); VESEY, David, R., GlaxoSmithKline, Harlow, Essex CM19 5AW (GB)
(74) Representative: Goddard, Carolyn Janice
(86) International application number: PCT/EP2004/014076
(87) International publication number: WO 2005/058915

(56) References cited:
- WO-A-01/70672
- WO-A-02/02505
- WO-A-98/33795
- WO-A-03/040096
- WO-A-20/04050619
- WO-A-20/04094430
- US-B1- 6 207 664
- KOZIKOWSKI A P ET AL: "Chemistry, binding affinities, and behavioral properties of a new class oF Antineophobic mitochondrial DBI receptor complex (mDRC) ligands" JOURNAL OF MEDICINAL CHEMISTRY, AMERICAN CHEMICAL SOCIETY. WASHINGTON, US, vol. 36, 1993, pages 2908-2920, XP002259612 ISSN: 0022-2623
- TETRAHEDRON, vol. 50, no. 7, 1994, pages 2017-2028, XP002319196

## Description

The present invention relates to novel hydroxyethylamine compounds having Asp2 (β-secretase, BACE1 or Memapsin) inhibitory activity, processes for their preparation, to compositions containing them and to their use in the manufacture of a medicament for the treatment of diseases characterised by elevated β- amyloid levels or β-amyloid deposits, particularly Alzheimer's disease.

Alzheimer's disease is a degenerative brain disorder in which extracellular deposition of Aβ in the form of senile plaques represents a key pathological hallmark of the disease (Selkoe, D. J. (2001) Physiological Reviews 81: 741-766). The presence of senile plaques is accompanied by a prominent inflammatory response and neuronal loss. β-amyloid (Aβ) exists in soluble and insoluble, fibrillar forms and a specific fibrillar form has been identified as the predominant neurotoxic species (Vassar, R. and Citron, M. (2000) Neuron 27: 419-422). In addition it has been reported that dementia correlates more closely with the levels of soluble amyloid rather than plaque burden (Naslund, J. et al. (2000) J. Am. Med. Assoc. 12: 1571-1577; Younkin, S. (2001) Nat. Med. 1: 8-19). Aβ is known to be produced through the cleavage of the beta amyloid precursor protein (also known as APP) by an aspartyl protease enzyme known as Asp2 (also known as β-secretase, BACE1 or Memapsin) (De Strooper, B. and Konig, G. (1999) Nature 402: 471-472).

Therefore, it has been proposed that inhibition of the Asp2 enzyme would reduce the level of APP processing and consequently reduce the levels of Aβ peptides found within the brain. Therefore, it is also thought that inhibition of the Asp2 enzyme would be an effective therapeutic target in the treatment of Alzheimer's disease.

APP is cleaved by a variety of proteolytic enzymes (De Strooper, B. and Konig, G. (1999) Nature 402: 471-472). The key enzymes in the amyloidogenic pathway are Asp2 (β-secretase) and γ-secretase both of which are aspartic proteinases and cleavage of APP by these enzymes generates Aβ. The non-amyloidogenic, α-secretase pathway, which precludes Aβ formation, has been shown to be catalysed by a number of proteinases, the best candidate being ADAM10, a disintegrin and metalloproteinase. Asp1 has been claimed to show both α- and β-secretase activity *in vitro*. The patterns of expression of Asp1 and Asp2 are quite different, Asp2 is most highly expressed in the pancreas and brain while Asp1 expression occurs in many other peripheral tissues. The Asp2 knockout mouse indicates that lack of Asp2 abolished Aβ production and also shows that in this animal model endogenous Asp1 cannot substitute for the Asp2 deficiency (Luo, Y. et al. (2001) Nat. Neurosci. 4: 231-232; Cal, H. et. al. (2001) Nat. Neurosci. 4: 233-234; Roberds, S. L. et al. (2001) Hum. Mol. Genet. 10: 1317-1324).

For an agent to be therapeutically useful in the treatment of Alzheimer's disease it is preferable that said agent is a potent inhibitor of the Asp2 enzyme, but should ideally also be selective for Asp2 over other enzymes of the aspartyl proteinase family, e.g. Cathepsin D (Connor, G. E. (1998) Cathepsin D in Handbook of Proteolytic Enzymes, Barrett, A. J., Rawlings, N. D., & Woesner, J. F. (Eds) Academic Press London. pp828-836).

WO 01/70672, WO 02/02512, WO 02/02505, WO 02/02506 and WO 03/040096 (Elan Pharmaceuticals Inc.) describe a series of hydroxyethylamine compounds having 3-secretase activity which are implicated to be useful in the treatment of Alzheimer's disease. US-B1-6207664 describes a series of squalene synthetase inhibitors which are claimed to be useful as hypocholesterolemic agents, hypotriglyceridemic agents, antiatherosclerosis agents, antifungal agents, anti-Alzheimer's agents or anti-acne agents.

We have found a novel series of compounds which are potent inhibitors of the Asp2 enzyme, thereby indicating the potential for these compounds to be effective in the treatment of disease characterised by elevated β-amyloid levels or β-amyloid deposits, such as Alzheimer's disease.

Thus, according to a first aspect of the present invention we provide a compound of formula (I): 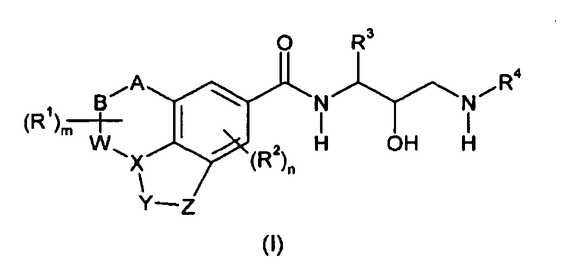 wherein
m represents 0;
n represents 0;
A-B represents -NR⁵-SO₂-;
R⁵ represents methyl or ethyl;
-W- represents -(CH₂)₂-;
X-Y-Z represents -C=CR⁸-NR⁹-;
R⁸ represents hydrogen;
R⁹ represents ethyl;
R³ represents unsubstituted benzyl;
R⁴ represents
   - C₁₋₁₀ alkyl optionally substituted by one or more halogen, C₁₋₆ alkoxy, haloC₁₋₆ alkoxy or -S-C₁₋₆ alkyl groups;
   - C₂₋₁₀ alkenyl optionally substituted by one or more C₁₋₆ alkyl groups;
   - C₃₋₁₀ alkynyl optionally substituted by one or more C₁₋₆ alkyl groups;
   - C₃₋₁₀ cycloalkyl optionally substituted by one or more halogen, C₁₋₆ alkyl or -C₂₋₆ alkynyl groups;
   - C₃₋₁₀ cycloalkenyl;
   - C₁₋₆ alkyl-C₃₋₁₀ cycloalkyl;
   - benzyl or phenyl, which benzyl or phenyl is optionally substituted by one or more halogen, cyano, haloC₁₋₆ alkoxy, haloC₁₋₆ alkyl, C₁₋₆ alkyl, C₁₋₆ alkoxy or -N(Me)₂ groups;
   - CH₂-pyrazolyl, - CH₂-pyridinyl, - CH₂-thienyl or - CH₂-isoxazolyl, which -CH₂-pyrazolyl, -CH₂-pyridinyl, -CH₂-thienyl or -CH₂-isoxazolyl is optionally substituted by one or more halogen, cyano, haloC₁₋₆ alkoxy, haloC₁₋₆ alkyl, C₁₋₆ alkyl or C₁₋₆ alkoxy groups;
   - C(R^{a}R^{b})-CONH-C₃₋₁₀ cycloalkyl ; or
   - tetrahydropyranyl optionally substituted by one or more C₁₋₆ alkyl groups; R^{a} and R^{b} independently represent hydrogen, methyl or together with the carbon atom to which they are attached form a cyclopropyl or cyclohexyl group;
or a pharmaceutically acceptable salt or solvate thereof.

The term 'C_{x-y} alkyl' as used herein as a group or a part of the group refers to a linear or branched saturated hydrocarbon group containing from x to y carbon atoms. Examples of such groups include methyl, ethyl, n-propyl, isopropyl, n-butyl, isobutyl, sec-butyl, tert butyl, n-pentyl, isopentyl, neopentyl or hexyl and the like.

The term 'C_{x-y} alkenyl' as used herein refers to a linear or branched hydrocarbon group containing one or more carbon-carbon double bonds and having from x to y carbon atoms. Examples of such groups include ethenyl, propenyl, butenyl, pentenyl or hexenyl and the like.

The term 'C_{x-y} alkynyl' as used herein refers to a linear or branched hydrocarbon group containing one or more carbon-carbon triple bonds and having from x to y carbon atoms. Examples of such groups include ethynyl, propynyl, butynyl, pentynyl or hexynyl and the like.

The term 'C_{x-y} alkoxy' as used herein refers to an -O-C_{x-y} alkyl group wherein C_{x-y} alkyl is as defined herein. Examples of such groups include methoxy, ethoxy, propoxy,'butoxy, pentoxy or hexoxy and the like.

The term 'C_{x-y} cycloalkyl' as used herein refers to a saturated monocyclic hydrocarbon ring of x to y carbon atoms. Examples of such groups include cyclopropyl, cyclobutyl, cyclopentyl, cyclohexyl, cycloheptyl or cyclooctyl and the like.

The term 'C_{x-y} cycloalkenyl' as used herein refers to an unsaturated non-aromatic monocyclic hydrocarbon ring of x to y carbon atoms containing one or more carbon-carbon double bonds. Examples of such groups include cyclopropenyl, cyclobutenyl, cyclopentenyl, cyclohexenyl, cycloheptenyl or cyclooctenyl and the like.

The term 'halogen' as used herein refers to a fluorine, chlorine, bromine or iodine atom.

The term 'haloC_{x-y} alkyl' as used herein refers to a C_{x-y} alkyl group as defined herein wherein at least one hydrogen atom is replaced with halogen. Examples of such groups include fluoroethyl, trifluoromethyl or trifluoroethyl and the like.

The term 'haloC_{x-y} alkoxy' as used herein refers to a C_{x-y} alkoxy group as herein defined wherein at least one hydrogen atom is replaced with halogen. Examples of such groups include difluoromethoxy or trifluoromethoxy and the like.

Preferably, R⁴ represents
-C₁₋₁₀ alkyl (e.g. ethyl, propyl, 1-methylpropyl, butyl, 3-methylbutyl, 2-ethylbutyl, 1 - propylbutyl, 3,3-dimethylbutyl, 1,5-dimethylhexyl or 1,1,5-trimethylhexyl) optionally substituted by one or more halogen (e.g. 2-fluoroethyl, 3-fluoropropyl, 2,2-difluoroethyl, 2,2,2-trifluoroethyl, 3,3,3-trifluoropropyl or 2,2,3,3,3-pentafluoropropyl), C₁₋₆ alkoxy (e.g. methoxy or propoxy), haloC₁₋₆ alkoxy (e.g. 2,2,2-trifluoroethoxy) or -S-C₁₋₆ alkyl (e.g. -S-methyl, -S-ethyl or -S-t-Bu) groups;
-C₂₋₁₀ alkenyl (e.g. propenyl or butenyl) optionally substituted by one or more C₁₋₆ alkyl groups (e.g. 2-methyl-2-propen-1-yl or 3-methyl-2-buten-1-yl);
-C₃₋₁₀ alkynyl (e.g. propynyl, butynyl or pentynyl) optionally substituted by one or more C₁₋₆ alkyl groups (e.g. 1,1-dimethyl-2-propyn-1-yl);
-C₃₋₁₀ cycloalkyl (e.g. cyclopropyl, cyclobutyl, cyclohexyl, cycloheptyl, cyclooctyl, tricyclodecyl or bicycloheptyl) optionally substituted by one or more halogen (e.g. fluorine), C₁₋₆ alkyl (e.g. methyl, ethyl or propyl) or C₂₋₆ alkynyl (e.g. ethynyl) groups;
-C₃₋₁₀ cycloalkenyl (e.g. cyclopentenyl);
-C₁₋₆ alkyl-C₃₋₁₀ cycloalkyl (e.g. -CH₂-cyclopropyl or -(CH₂)₂-cyclohexyl);
benzyl or phenyl optionally substituted by one or more halogen (e.g. chlorine), cyano, haloC₁₋₆ alkoxy (e.g. -OCF₃), haloC₁₋₆ alkyl (e.g. -CF₃), C₁₋₆ alkyl (e.g. methyl), C ₁₋₆ alkoxy (e.g. methoxy) or -NR²²R²³ (e.g. -N(Me)₂) groups;
-CH₂-pyrazolyl, -CH₂-pyridinyl, -CH₂-thienyl or -CH₂-isoxazolyl optionally substituted by one or more halogen, cyano, haloC₁₋₆ alkoxy (e.g. -OCF₃), haloC₁₋₆ alkyl (e.g. -CF₃ or trifluoroethyl), C₁₋₆ alkyl (e.g. methyl or ethyl) or C₁₋₆ alkoxy (e.g. methoxy) groups;
-C(R^{a}R^{b})-CONH-C₃₋₁₀ cycloalkyl (e.g. -C(R^{a}R^{b})-CONH-cyclohexyl); or
tetrahydropyranyl optionally substituted by one or more C₁₋₈ alkyl (e.g. methyl) groups.

Preferably, R^{a} and R^{b} both represent hydrogen.

Preferred compounds according to the invention include examples E3-43, E46-58, E60-65 and E70-90 as shown below, or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic or organic acids e.g. hydrochlorides, hydrobromides, sulphates, phosphates, acetates, benzoates, citrates, nitrates, succinates, lactates, tartrates, fumarates, maleates, 1-hydroxy-2-naphthoates, palmoates, methanesulphonates, p-toluenesulphonates, naphthalenesulphonates, formates or trifluoroacetates. The present invention includes within its scope all possible stoichiometric and non-stoichiometric forms.

The compounds of formula (I) may be prepared in crystalline or non-crystalline form, and, if crystalline, may optionally be solvated, e.g. as the hydrate. This invention includes within its scope stoichiometric solvates (e.g. hydrates) as well as compounds containing variable amounts of solvent (e.g. water).

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof. Preferably, compounds of formula (I) are in the form of a single enantiomer of formula (Ia):

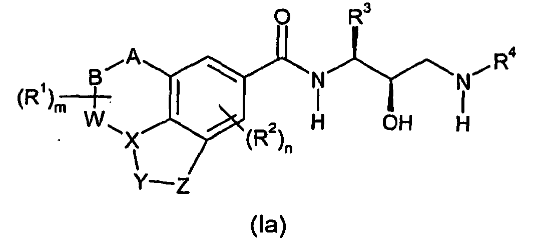

The compounds of formula (I) and salts and solvates thereof may be prepared by the methodology described hereinafter, constituting a further aspect of this invention.

A process according to the invention for preparing a compound of formula (I) which comprises:
(a) reacting a compound of formula (II) 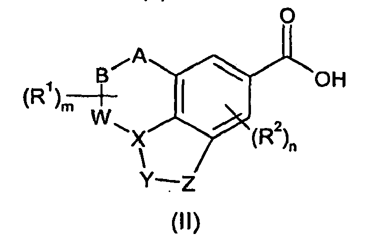 or an activated and/or optionally protected derivative thereof wherein R¹, R², m, n, A, B, W, X, Y and Z are as defined above, with a compound of formula (III) 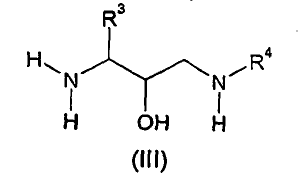 wherein R³ and R⁴ are as defined above; or
(b) preparing a compound of formula (I) which comprises reductive alkylation of a compound of formula (IV) 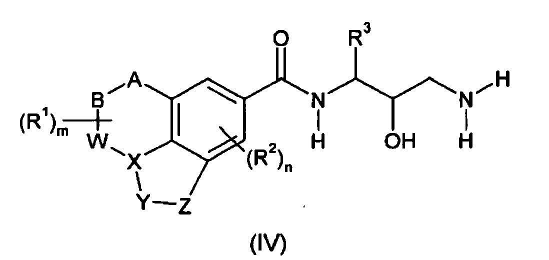 wherein R¹, R², R³, m, n, A, B, W, X, Y and Z are as defined above, with an appropriate aldehyde or ketone; or
(c) deprotecting a compound of formula (I) which is protected; and optionally thereafter
(d) interconversion of compounds of formula (I) to other compounds of formula (I).

Where the compound of formula (II) is an activated derivative, (e.g. by activation of a carboxylic acid to an acid chloride, mixed anhydride, active ester, O-acyl-isourea or other species), process (a) typically comprises treatment of said activated derivative with an amine (Ogliaruso, M.A.; Wolfe, J.F. in The Chemistry of Functional Groups (Ed. Patai, S.) Suppl. B: The Chemistry of Acid Derivatives, Pt. 1 (John Wiley and Sons, 1979), pp 442-8; Beckwith, A.L.J. in The Chemistry of Functional Groups (Ed. Patai, S.) Suppl. B: The Chemistry of Amides (Ed. Zabricky, J.) (John Wiley and Sons, 1970), p 73 ff) The acid of formula (II) and amine are preferably reacted in the presence of activating agents such as 1-(dimethylaminopropyl)-3-ethylcarbodiimide hydrochloride (EDC) and 1-hydroxybenzotriazole (HOBT), or O-(7-azabenzotriazol-1-yl)-*N*,*N*,*N'*,*N'-*tetramethyluronium hexafluorophosphate (HATU).

Where the compound of formula (II) is a carboxylic acid, process (a) typically comprises the use of water soluble carbodiimide, HOBT and a suitable base such as tertiary alkylamine or pyridine in a suitable solvent such as DMF and at a suitable temperature, e.g. between 0°C and room temperature.

Process (b) typically comprises the use of sodium borohydride triacetate in the presence of a suitable solvent, such as ethanol, dichloromethane and 1,2-dichloroethane and at a suitable temperature, e.g. between 0°C and room temperature.

In process (c), examples of protecting groups and the means for their removal can be found in T. W. Greene and P.G.M. Wuts 'Protective Groups in Organic Synthesis' (J. Wiley and Sons, 3rd Ed. 1999). Suitable amine protecting groups include aryl sulphonyl (e.g. tosyl), acyl (e.g. acetyl), carbamoyl (e.g. benzyloxycarbonyl or t-butoxycarbonyl) and arylalkyl (e.g. benzyl), which may be removed by hydrolysis or hydrogenolysis as appropriate. Other suitable amine protecting groups include trifluoroacetyl (-COCF₃) which may be removed by base catalysed hydrolysis. Suitable hydroxy protecting groups would be silyl based groups such as t-butyldimethylsilyl, which may be removed using standard methods, for example use of an acid such as trifluoroacetic or hydrochloric acid or a fluoride source such as tetra n-butylammonium fluoride.

Process (d) may be performed using conventional interconversion procedures such as epimerisation, oxidation, reduction, alkylation, aromatic substitution, ester hydrolysis, amide bond formation or removal and sulphonylation. For example, compounds of formula (I) wherein W represents -C(H)=C(H)- or -CH₂-C(H)=C(H)- may be converted to compounds of formula (I) wherein W represents -(CH₂)₂- or -(CH₂)₃- by catalytic hydrogenation compounds as herein described.

Compounds of formula (II) and/or activated and optionally protected derivatives thereof wherein W represents -C(H)=C(H)- or -CH₂-C(H)=C(H)- may be prepared in accordance with the following process: 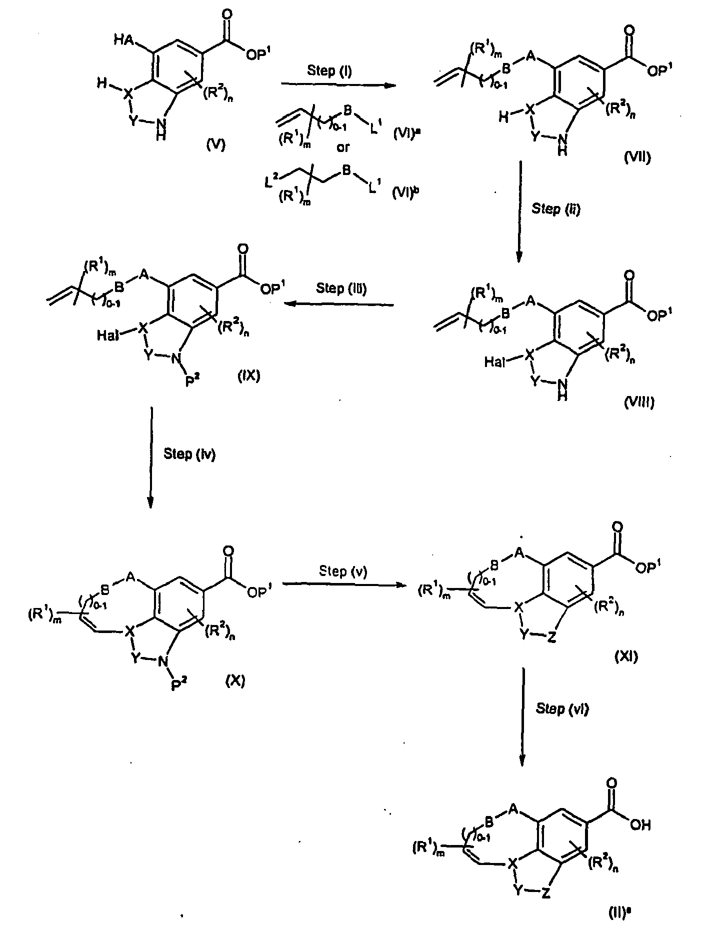 wherein R¹, R², m, n, A, B, X, Y and Z are as defined above, P¹ represents a suitable group such as C₁₋₆ alkyl, P² represents a suitable group such as -COC₁₋₆ alkyl, -CO₂C₁₋₆ alkyl or -SO₂aryl, L¹ and L² independently represent a suitable leaving group such as a halogen atom (e.g. chlorine) and Hal represents a halogen atom, such as bromine or iodine.

Step (i) typically comprises reaction of a compound of formula (V) with a compound of formula (VI)^{a} or (VI)^{b} in the presence of a suitable base such as pyridine in the presence of a suitable reagent, e.g. DMAP and a suitable solvent such as dichloromethane at a suitable temperature, such as room temperature.

Step (ii) typically comprises the use of a halogen such as bromine in the presence of a suitable solvent such as dimethylformamide at a suitable temperature, such as room temperature.

Step (iii) typically comprises introduction of an N-protecting group using standard protocols. For example, an acetate group can be introduced by treatment with acetic anhydride in the presence of a suitable solvent such as pyridine at a suitable temperature, such as room temperature.

Step (iv) typically comprises a standard procedure for addition of a vinyl halide to an alkene, such as the use of a mixture of tetrabutylammonium chloride, palladium acetate and triorthotolyl phosphine in an appropriate solvent such as tetrahydrofuran at an appropriate temperature such as 65°C.

Step (v) typically comprises the use of standard deprotection conditions (e.g. treatment with a suitable amine such as triethylamine in a suitable solvent such as ethanol at an appropriate temperature such as 80°C) and subsequent derivatisation of Z using standard methods (e.g. treatment with a base such as sodium hydride and an alkylating agent such as ethyl iodide in a suitable solvent such as dimethylformamide at an appropriate temperature such as room temperature).

Step (vi) typically comprises a standard procedure for conversion of a carboxylic ester to an acid, such as the use of an appropriate hydroxide salt such as lithium or sodium salt in an appropriate solvent such as methanol at an appropriate temperature such as 50°C. In the case of a tert-butyl ester this conversion can be achieved by the use of an appropriate acid such as trifluoroacetic acid in an appropriate solvent such as dichloromethane at an appropriate temperature such as 0°C. Activated derivatives of compounds of formula (II) may then be prepared as described in process (a) above.

Compounds of formula (II) wherein W represents -(CH₂)₂- or -(CH₂)₃- may be prepared in an identical manner to the process described above except an additional step is required in which compounds of formula (XI) are hydrogenated prior to step (vi). This step, typically comprises the use of standard reducing conditions such as treatment with 10% palladium on charcoal and ammonium formate in a suitable solvent such as methanol at a suitable temperature e.g. reflux.

Compounds of formula (II) wherein W represents -C(H)=C(H)-, A-B represents -NR⁵-SO₂- and m represents 0 may also be prepared in accordance with the following process: 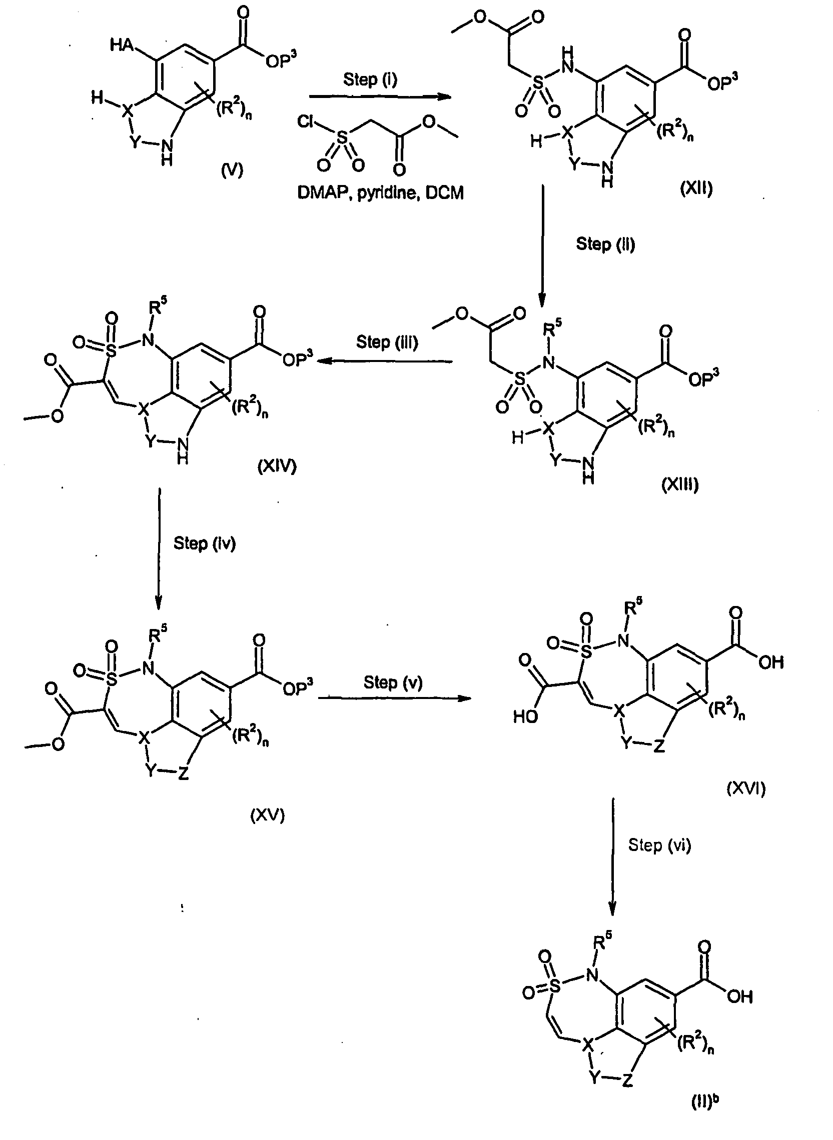 wherein R², n, R⁵, X, Y and Z are as defined above and P³ represents a suitable group such as C₁₋₆ alkyl.

Step (i) typically comprises reaction of a compound of formula (V) with methyl (chlorosulfonyl)acetate in the presence of a suitable base such as pyridine in the presence of a suitable reagent, e.g. DMAP and a suitable solvent such as dichloromethane at a suitable temperature, such as room temperature.

Step (ii) typically comprises reaction with an alkyl halide such as iodomethane in the presence of a suitable base such as potassium carbonate and a suitable solvent such as dimethylformamide at a suitable temperature such as room temperature.

Step (iii) typically comprises reaction with a reagent prepared by mixing together phosphorus oxychloride and dimethylformamide in the presence of a suitable solvent such as dimethylformamide at a suitable temperature, such as 60°C.

Step (iv) typically comprises reaction with an alkyl halide such as iodoethane in the presence of a suitable base such as sodium hydride and a suitable solvent such as dimethylformamide at a suitable temperature such as room temperature.

Step (v) typically comprises a standard procedure for conversion of a carboxylic ester to an acid, such as the use of an appropriate hydroxide salt like lithium or sodium salt in an appropriate solvent such as methanol at an appropriate temperature such as 65°C

Step (vi) typically comprises a standard procedure for decarboxylation such as treatment with an acid such as hydrogen chloride in a suitable solvent such as dioxane at a suitable temperature such as 100°C. Activated derivatives of compounds of formula (II) may then be prepared as described in process (a) above.

Compounds of formula (III) may be prepared in accordance with the following process: 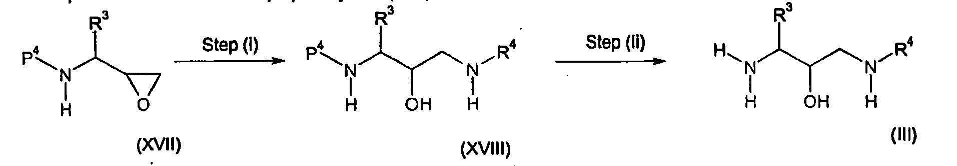 wherein R³ and R⁴ are as defined above and P⁴ represents a suitable amine protecting group, such as t-butoxycarbonyl.

Step (i) typically comprises the reaction of a compound of formula (XVII) with a compound of formula NH₂R⁴ in the presence of a suitable solvent, e.g. ethanol at a suitable temperature, e.g. reflux.

Step (ii) typically comprises the use of suitable deprotection reactions as described above for process (c), e.g. when P⁴ represents t-butoxycarbonyl, deprotection typically comprises the use of trifluoroacetic acid in the presence of a suitable solvent, such as dichloromethane at a suitable temperature, e.g. between 0°C and room temperature.

Compounds of formula (IV) may be prepared in accordance with the following process: 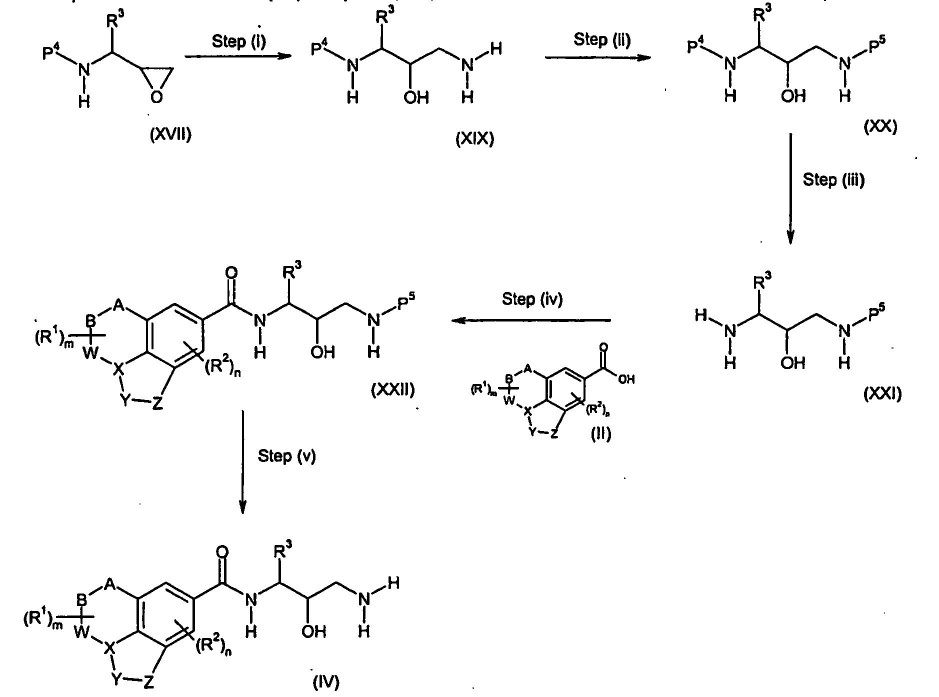 wherein R¹, R², R³, m, n, A, B, W, X, Y, Z and P⁴ are as defined above and P⁵ represents a suitable amine protecting group different to P⁴, such as -COOCH₂-phenyl.

Step (i) typically comprises the reaction of a compound of formula (XVII) in aqueous ammonia in the presence of a suitable solvent, e.g. ethanol at a suitable temperature, e.g. reflux.

When P⁵ represents -COOCH₂-phenyl, step (ii) typically comprises the use of ClCOOCH₂-phenyl in the presence of a suitable base, e.g. triethylamine, a suitable solvent, e.g. dimethylformamide at a suitable temperature, e.g. between 0°C and room temperature.

Step (iii) typically comprises the use of suitable deprotection reactions as described above for process (c), e.g. when P⁴ represents t-butoxycarbonyl, deprotection typically comprises the use of trifluoroacetic acid in the presence of a suitable solvent, such as dichloromethane at a suitable temperature, e.g. between 0°C and room temperature.

Step (iv) typically comprises reacting a compound of formula (XXI) with a compound of formula (II) in the presence of water soluble carbodiimide and HOBT.

Step (v) typically comprises the use of suitable deprotection reactions as described above for process (c), e.g. when P⁵ represents -COOCH₂-phenyl, deprotection typically comprises the use of a suitable catalyst, e.g. palladium in the presence of a suitable solvent, e.g. water and ethanol and in the presence of a suitable hydrogen source, e.g. ammonium formate at a suitable temperature, e.g. 60°C.

Compounds of formula (V)**,** (VI)^{a}, (VI)^{b} and (XVII) are either commercially available or may be prepared from commercially available compounds using standard procedures.

As a further aspect of the invention there is thus provided a compound of formula (I) or a pharmaceutically acceptable salt or solvate thereof for use as a pharmaceutical, particularly in the treatment of patients with diseases characterised by elevated β-amyloid levels or β-amyloid deposits.

According to another aspect of the invention, there is provided the use of a compound of formula (I) or a physiologically acceptable salt or solvate thereof for the manufacture of a medicament for the treatment of patients with diseases characterised by elevated β-amyloid levels or β-amyloid deposits.

Compounds of formula (I) may also be useful in a method for the treatment of a human or animal subject with diseases characterised by elevated β-amyloid levels or β-amyloid deposits, which method comprises administering to said human or animal subject an effective amount of a compound of formula (I) or a physiologically acceptable salt or solvate thereof.

As a further aspect of the invention there is thus provided a pharmaceutical composition comprising a compound of formula (I) or a pharmaceutically acceptable salt or solvate thereof for use in the treatment of diseases characterised by elevated β-amyloid levels or β-amyloid deposits.

It will be appreciated by those skilled in the art that reference herein to treatment extends to prophylaxis as well as the treatment of diseases characterised by elevated β-amyloid levels or β-amyloid deposits.

The compounds according to the invention may be formulated for administration in any convenient way, and the invention therefore also includes within its scope pharmaceutical compositions for use in the therapy of diseases characterised by elevated β-amyloid levels or β-amyloid deposits, comprising a compound of formula (I) or a physiologically acceptable salt or solvate thereof together, if desirable, with one or more physiologically acceptable diluents or carriers.

It will be appreciated that diseases characterised by elevated β-amyloid levels or β-amyloid deposits include Alzheimer's disease, mild cognitive impairment, Down's syndrome, hereditary cerebral haemorrhage with β-amyloidosis of the Dutch type, cerebral β-amyloid angiopathy and various types of degenerative dementias, such as those associated with Parkinson's disease, progressive supranuclear palsy, cortical basal degeneration and diffuse Lewis body type of Alzheimer's disease.

Most preferably, the disease characterised by elevated β-amyloid levels or β-amyloid deposits is Alzheimer's disease.

There is also provided a process for preparing such a pharmaceutical formulation which comprises mixing the ingredients.

Compounds of formula (I) may be used in combination with other therapeutic agents. Suitable examples of such other therapeutic agents may be acetylcholine esterase inhibitors (such as tetrahydroaminoacridine, donepezil hydrochloride and rivastigmine), gamma secretase inhibitors, anti-inflammatory agents (such as cyclooxygenase II inhibitors), antioxidants (such as Vitamin E and ginkolidesor), statins or p-glycoprotein (P-gp) inhibitors (such as cyclosporin A, verapamil, tamoxifen, quinidine, Vitamin E-TGPS, ritonavir, megestrol acetate, progesterone, rapamycin, 10,11-methanodibenzosuberane, phenothiazines, acridine derivatives such as GF120918, FK506, VX-710, LY335979, PSC-833, GF-102 and 918).

The compounds according to the invention may, for example, be formulated for oral, inhaled, intranasal, buccal, enteral, parenteral, topical, sublingual, intrathecal or rectal administration, preferably for oral administration.

Tablets and capsules for oral administration may contain conventional excipients such as binding agents, for example syrup, acacia, gelatin, sorbitol, tragacanth, mucilage of starch, cellulose or polyvinyl pyrrolidone; fillers, for example, lactose, microcrystalline cellulose, sugar, maize- starch, calcium phosphate or sorbitol; lubricants, for example, magnesium stearate, stearic acid, talc, polyethylene glycol or silica; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use: Such liquid preparations may contain conventional additives such as suspending agents, for example, sorbitol syrup, methyl cellulose, glucose/sugar syrup, gelatin, hydroxymethyl cellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example almond oil, fractionated coconut oil, oily esters, propylene glycol or ethyl alcohol; or preservatives, for example, methyl or propyl p-hydroxybenzoates or sorbic acid. The preparations may also contain buffer salts, flavouring, colouring and/or sweetening agents (e.g. mannitol) as appropriate.
For buccal administration the compositions may take the form of tablets or lozenges formulated in conventional manner.

The compounds may also be formulated as suppositories, e.g. containing conventional suppository bases such as cocoa butter or other glycerides.

The compounds according to the invention may also be formulated for parenteral administration by bolus injection or continuous infusion and may be presented in unit dose form, for instance as ampoules, vials, small volume infusions or pre-filled syringes, or in multi-dose containers with an added preservative. The compositions may take such forms as solutions, suspensions, or emulsions in aqueous or non-aqueous vehicles, and may contain formulatory agents such as anti-oxidants, buffers, antimicrobial agents and/or tonicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

When the compounds of the invention are administered topically they may be presented as a cream, ointment or patch.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 3000 mg; and such unit doses may be administered more than once a day, for example one, two, three or four times per day (preferably once or twice); and such therapy may extend for a number of weeks, months or years.

### Examples

### Preparation of Intermediates

### Description 1

### Methyl 4-methyl-3,5-dinitrobenzoate (D1)

Thionyl chloride (72 g, 615 mmol) was added dropwise, with stirring, to a suspension of 4-methyl-3,5-dinitrobenzoic acid (commercially available from Aldrich)(100 g, 440 mmol) in methanol (300 ml). The resulting solution was left to stand at room temperature overnight and the precipitate that formed was then collected by filtration. The filtrate was washed with cold methanol to give the title compound (D1) as a white solid (104 g, 430 mmol) which was used in the next step without further purification.

### Description 2

### Methyl 4-[(E)-2-(dimethylamino)ethenyl]-3,5-dinitrobenzoate (D2)

A solution of methyl 4-methyl-3,5-dinitrobenzoate (D1) (40 g, 170 mmol) in dimethylformamide (50 ml) was treated with N,N-dimethylformamide dimethyl acetal (50 ml, 380 mmol) and the resulting mixture was heated at 50°C for 1 h. The solvent was then evaporated and the residue was triturated with diethyl ether/i-hexane (1:1) to give crude title compound (D2) (40 g, 136 mmol) as a dark red solid. This was used in subsequent reactions without further purification.

### Description 3

### Methyl 4-amino-1H-indole-6-carboxylate (D3)

Methyl 4-[(*E*)-2-(dimethylamino)ethenyl]-3,5-dinitrobenzoate (D2) (10.0 g, 34 mmol) in methanol (150 ml) was treated with ammonium formate (21.4 g, 340 mmol) and wet (50% water) 10% palladium on carbon (3 g) under a nitrogen atmosphere. The mixture was then heated at 50°C for 1 h. The mixture was filtered and the solvent was removed by evaporation. The residue was dissolved in ethyl acetate (200 ml) and washed with saturated aqueous sodium hydrogen carbonate (100 ml). The organic phase was then dried over magnesium sulfate, filtered and concentrated *in vacuo*. The residue was triturated with diethyl ether/i-hexane (1:1) to give the title compound (D3) (5.0 g, 26 mmol) as a pale pink solid which was used in subsequent reactions without further purification. [M+H]⁺ = 191.1, RT = 2.17 min.

### Description 4

### Methyl 4-[(ethenylsulfonyl)amino]-1H-indole-6-carboxylate (D4)

To a solution of methyl 4-amino-1*H*-indole-6-carboxylate (D3)(2.0 g, 10.5 mmol) in dichloromethane (100 ml) was added triethylamine (2.13 g, 21 mmol) and the mixture was heated gently to dissolve any remaining solids. 2-Chloro-1-ethane sulfonyl chloride (1.63 g, 10 mmol) was then added dropwise to the mixture and stirring continued for 30 min. At this point a further quantity of 2-chloro-1-ethane sulfonyl chloride (0.39 g, 2.4 mmol) was added and stirring continued for a further 30 min. The mixture was washed sequentially with 2M aqueous hydrogen chloride (50 ml) and saturated aqueous sodium hydrogen carbonate (50 ml) and then the organic phase was dried over magnesium sulfate, filtered and evaporated *in vacuo*. The residue was triturated with diethyl ether/i-hexane (1:1) to give crude title compound (D4)(1.6 g, 5.7 mmol) as a brown solid which was used in subsequent reactions without further purification. [M+H]⁺ = 281.1, RT = 2.23 min.

### Description 5

### Methyl 4-[(ethenylsulfonyl)(methyl)amino]-1H-indole-6-carboxylate (D5)

A solution of methyl 4-[(ethenylsulfonyl)amino]-1*H*-indole-6-carboxylate (D4)(5.0 g, 17.9 mmol) in dimethylformamide (50 ml) was treated with potassium carbonate (2.48 g, 18 mmol) and iodomethane (1.12 ml, 18 mmol) at room temperature for 90 min. Diethyl ether (200 ml) was added to the mixture and the mixture was then washed sequentially with 2M aqueous hydrogen chloride (100 ml), saturated aqueous sodium hydrogen carbonate (100 ml) and water (3 x 100 ml). The aqueous phase was then dried over magnesium sulfate and then filtered and evaporated *in vacuo* to give the title compound (D5)(4.5 g, 15.3 mmol) as a brown foam. This was used without further purification in subsequent reactions. [M+H]⁺ = 295.1, RT = 2.48 min.

### Description 6

### Methyl 4-[(ethenylsulfonyl)(ethyl)amino]-1H-indole-6-carboxylate (D6)

Methyl 4-[(ethenylsulfonyl)(ethyl)amino]-1*H*-indole-6-carboxylate (D6) was obtained in an analogous manner to that described for the synthesis of (D5) but using iodoethane in the place of iodomethane. [M+H]⁺ = 309.1, RT = 2.65 min.

### Description 7

### Methyl 3-bromo-4-[(ethenylsulfonyl)(methyl)amino]-1H-indole-6-carboxylate (D7)

A solution of methyl 4-[(ethenylsulfonyl)(methyl)amino]-1*H*-indole-6-carboxylate (D5)(0.700 g, 2.4 mmol) in dimethylformamide (20 ml) was treated dropwise with a solution of bromine (0.12 ml, 2.3 mmol) in dimethylformamide (5 ml) over 15 min. The solvent was then evaporated *in vacuo* and the residue taken up in ethyl acetate (50 ml) and washed with water (2 x 50 ml). The organic phase was then dried over magnesium sulfate, filtered and evaporated to give the title compound (D7)(0.800 g, 2.2 mmol) as a pale brown foam. [M+H]⁺ = 373.0, RT = 2.74 min.

### Description 8

### Methyl 3-bromo-4-[(ethenylsulfonyl)(ethyl)amino]-1H-indole-6-carboxylate (D8)

Methyl 3-bromo-4-[(ethenylsulfonyl)(ethyl)amino]-1*H*-indole-6-carboxylate (D8) was obtained in an analogous manner to that described for the synthesis of (D7) but using methyl 4-[(ethenylsulfonyl)(ethyl)amino]-1*H*-indole-6-carboxylate (D6) in the place of methyl 4-[(ethenylsulfonyl)(methyl)amino]-1*H*-indole-6-carboxylate (D5). [M+H]⁺ = 389.1, RT = 2.89 min.

### Description 9

### Methyl 1-acetyl-3-bromo-4-[(ethenylsulfonyl)(methyl)amino]-1H-indole-6-carboxylate (D9)

A solution of methyl 3-bromo-4-[(ethenylsulfonyl)(methyl)amino]-1*H*-indole-6-carboxylate (D7)(0.800 g, 2.2 mmol) in pyridine (5 ml) was treated with acetic anhydride (1 ml, 10.6 mmol) and the resulting mixture was stirred overnight at room temperature. The mixture was diluted with ethyl acetate (50 ml) and washed sequentially with 2M aqueous hydrogen chloride (50 ml) and saturated aqueous sodium hydrogen carbonate (50 ml). The organic phase was dried over magnesium sulfate and then filtered and evaporated *in vacuo*. The crude product was recrystallised from ethyl acetate/i-hexane to obtain the title compound (D9)(0.510 g, 1.23 mmol) as a pink solid. [M+H]⁺ = 417.0, RT = 2.85 min.

### Description 10

### Methyl 1-acetyl-3-bromo-4-[(ethenylsulfonyl)(ethyl)amino]-1H-indole-6-carboxylate (D10)

Methyl-1-acetyl-3-bromo-4-[(ethenylsulfonyl)(ethyl)amino]-1*H*-indole-6-carboxylate (D10) was obtained in an analogous manner to that described for the synthesis of (D9) but using methyl 3-bromo-4-[(ethenylsulfonyl)(ethyl)amino]-1*H*-indole-6-carboxylate (D8) in the place of methyl 3-bromo-4-[(ethenylsulfonyl)(methyl)amino]-1*H*-indole-6-carboxylate (D7). RT = 3.01 min

### Description 11

### Methyl 1-ethyl-1,6-dihydro[1,2]thiazepino[5,4,3-cd] indole-8-carboxylate 2,2-dioxide (D11)

A solution of methyl-1-acetyl-3-bromo-4-[(ethenylsulfonyl)(ethyl)amino]-1*H*-indole-6-carboxylate (D10)(0.400 g, 0.94 mmol) in tetrahydrofuran (30 ml) was treated with tetrabutylammonium chloride (0.560 g, 2.0 mmol), palladium diacetate (0.220 g, 1.0 mmol), and triorthotolyl phosphine (0.304 g, 2.0 mmol) under a nitrogen atmosphere. The mixture was heated at reflux for 30 min. The solvent was evaporated and the residue was dissolved in ethyl acetate (100 ml) and washed sequentially with 2M aqueous hydrogen chloride (50 ml) and saturated aqueous sodium hydrogen carbonate (50 ml). The organic phase was dried over magnesium sulfate and then filtered and evaporated *in vacuo*. The residue was now dissolved in ethanol (50 ml) and treated with triethylamine (0.5 ml, 3.5 mmol). The mixture was then heated at reflux for 15 min before cooling and workup as described above gave the crude product which was crystallised from ethyl acetate/i-hexane to give the title compound (D11)(0.280 g, 0.92 mmol) as a brown solid which was used in subsequent reactions without further purification. [M+H]⁺ = 307.1, RT = 2.54 min

### Description 12

### Methyl 1-methyl-1,6-dihydro[1,2]thiazepino[5,4,3-cd]indole-8-carboxylate 2,2-dioxide (D12)

Methyl 1-methyl-1,6-dihydro[1,2]thiazepino[5,4,3-*cd*]indole-8-carboxylate 2,2-dioxide (D12) was obtained in an analogous manner to that described for the synthesis of (D11) but using methyl 1-acetyl-3-bromo-4-[(ethenylsulfonyl)(methyl)amino]-1*H*-indole-6-carboxylate (D9) in the place of methyl-1-acetyl-3-bromo-4-[(ethenylsulfonyl)(ethyl)amino]-1*H*-indole-6-carboxylate (D10). [M+H]⁺ = 293.1 ,RT = 2.37 min

### Description 13

### Methyl 1-methyl-1,3,4,6-tetrahydro[1,2]thiazepino[5,4,3-cd]indole-8-carboxylate 2,2-dioxide (D13)

A solution of methyl 1-methyl-1,6-dihydro[1,2]thiazepino[5,4,3-*cd*]indole-8-carboxylate 2,2-dioxide (D12)(0.400 g, 1.37 mmol) in methanol (50 ml) was treated with ammonium formate (0.800 g, 12.7 mmol) and 10% palladium on charcoal (0.4 g) and the mixture was heated at reflux for 3.5 h. The mixture was then filtered and evaporated *in vacuo.* The residue was dissolved in ethyl acetate (100 ml) and washed with saturated aqueous sodium hydrogen carbonate (50 ml) then dried over magnesium sulfate. Filtration and evaporation *in vacuo* gave the title compound (D13)(0.220 g, 0.75 mmol) as a brown solid. This was used in subsequent reactions without further purification. [M+H]⁺= 295.1, RT = 2.32 min

### Description 14

### Methyl 4-({[2-(methyloxy)-2-oxoethyl]sulfonyl}amino)-1H-indole-6-carboxylate (D14)

A solution of methyl 4-amino-1*H*-indole-6-carboxylate (D3) (9 g, 47 mmol) in dichloromethane (180 ml) was treated with pyridine (5.8 ml) and DMAP (0.577 g) and then methyl (chlorosulfonyl)acetate [56146-83-9] (8.63 g, 50 mmol) was added dropwise. The resulting black mixture was stirred at room temperature overnight. An additional quantity of methyl (chlorosulfonyl)acetate (1.2 g) was added and the mixture stirred for a further 48 hours at room temperature. The mixture was diluted with ethyl acetate and washed sequentially with saturated aqueous sodium hydrogen carbonate and brine, dried over anhydrous sodium sulfate and concentrated *in vacuo*. The resulting solid was triturated with ether to give the crude title compound (D14) as a brown solid (9.37 g). [M-H]⁻ = 325.2, RT = 2.14 min

### Description 15

### Methyl 4-(methyl{[2-(methyloxy)-2-oxoethyl]sulfonyl}amino)-1H-indole-6-carboxylate (D15)

A solution of methyl 4-({[2-(methyloxy)-2-oxoethyl]sulfonyl}amino)-1*H*-indole-6-carboxylate (D14) (13.4 g, 41.1 mmol) in dimethylformamide (145 ml) was treated with potassium carbonate (19.8 g) and iodomethane (2.6 ml) and stirred overnight at room temperature. The mixture was evaporated and the residue diluted with ethyl acetate and washed with saturated aqueous sodium hydrogen carbonate then the ethyl acetate layer was dried over magnesium sulfate and concentrated *in vacuo*. The crude material was purified by biotage (eluting with ethyl acetate:hexane) and the resulting solid was triturated with ether to give the title compound (D15) as an orange solid (4.93 g). [M-H]⁻ = 339.2, RT = 2.38 min

### Description 16

### Dimethyl 1-methyl-1,6-dihydro[1,2]thiazepino[5,4,3-cd]indole-3,8-dicarboxylate 2,2-dioxide (D16)

Phosphorus oxychloride (1.4 ml) was added dropwise to dimethylformamide (4.5 ml) at 0°C and the mixture was stirred for a further 15 minutes. The mixture was then treated with a solution of methyl 4-(methyl{[2-(methyloxy)-2-oxoethyl]sulfonyl}amino)-1*H*-indole-6-carboxylate (D15) (4.93 g, 14.5 mmol) in dimethylformamide (18 ml) and heated at 50°C for 1h. A further amount of phosphorus oxychloride (0.7 ml) was added to the mixture and heating continued overnight at 60°C. The mixture was cooled and any excess phosphorus oxychloride and dimethylformamide were removed by evaporation. The residue was diluted carefully with dichloromethane (500 ml) and water (200 ml) and 2N aqueous sodium hydroxide was added until the pH of the aqueous layer was 7. Any solid that precipitated was collected by filtration and set aside. The dichloromethane layer was separated and then washed with water and dried over anhydrous sodium sulphate. Filtration and evaporation of the dichloromethane layer gave a yellow solid which was combined with the solid set aside previously. The solid was washed with dichloromethane and ether to give the title compound (D16) as a yellow solid ( 2.85g). [M+H]⁺ = 351.1, RT = 2.27 min

### Description 17

### Dimethyl 6-ethyl-1-methyl-1,6-dihydro[1,2]thiazepino[5,4,3-cd]indole-3,8-dicarboxylate 2,2-dioxide (D17)

A solution of dimethyl 1-methyl-1,6-dihydro[1,2]thiazepino[5,4,3-*cd*]indole-3,8-dicarboxylate 2,2-dioxide (D16) (2.67 g, 7.63 mmol) in dimethylformamide (11 ml) was treated with sodium hydride (0.305 g, 60% suspension in oil) and stirred at room temperature for 10 minutes. The mixture was then treated with ethyl iodide (1.22 ml) and stirred overnight at room temperature. The mixture was diluted with ethyl acetate and washed with saturated aqueous sodium hydrogen carbonate and then with brine. The solid that precipitated at this stage was collected by filtration and washed with ether and water and then set aside. The remaining organic fraction was dried over magnesium sulfate and concentrated *in vacuo*. The crude material was purified by trituration with ether and the resulting solid was combined with the solid collected earlier to give the title compound (D17) as a yellow solid (2.57 g). [M+H]⁺ = 379.1, RT = 2.61 min

### Description 18

### Dimethyl 1-methyl-6-(1-methylethyl)-1,6-dihydro[1,2]thiazepino[5,4,3-cd]indole-3,8-dicarboxylate 2,2-dioxide (D18)

In a manner analogous to that described for the preparation of compound (D17), but using 2-iodopropane in the place of iodoethane, dimethyl 1-methyl-1,6-dihydro[1,2]thiazepino[5,4,3-*cd*]indole-3,8-dicarboxylate 2,2-dioxide (D16) was reacted to give the title compound (D18) as a yellow solid. [M+H]⁺ = 391.2, RT = 2.86 min

### Description 19

### Dimethyl 1-methyl-6-propyl-1,6-dihydro[1,2]thiazepino[5,4,3-cd]indole-3,8-dicarboxylate 2,2-dioxide (D19)

In a manner analogous to that described for the preparation of compound (D17), but using 1-bromopropane in the place of iodoethane, dimethyl 1-methyl-1,6-dihydro[1,2]thiazepino[5,4,3-*cd*]indole-3,8-dicarboxylate 2,2-dioxide (D16) was reacted to give the title compound (D19) as a yellow solid. [M+H]⁺ = 393.2, RT = 2.87 min

### Description 20

### Dimethyl 6-butyl-1-methyl-1,6-dihydro[1,2]thiazepino[5,4,3-cd]indole-3,8-dicarboxylate 2,2-dioxide (D20)

In a manner analogous to that described for the preparation of compound (D17), but using 1-iodobutane in the place of iodoethane, dimethyl 1-methyl-1,6-dihydro[1,2]thiazepino[5,4,3-*cd*]indole-3,8-dicarboxylate 2,2-dioxide (D16) was reacted to give the title compound (D20) as a yellow solid. [M+Na]⁺ = 429.18, RT = 3.04 min

### Description 21

### 6-Ethyl-1-methyl-1,6-dihydro[1,2]thiazepino[5,4,3-cd]indole-3,8-dicarboxylic acid 2,2-dioxide (D21)

Dimethyl 6-ethyl-1-methyl-1,6-dihydro[1,2]thiazepino[5,4,3-*cd*]indole-3,8-dicarboxylate 2,2-dioxide (D17) (2.57 g, 6.79 mmol) was dissolved in methanol (50 ml) and treated with 2N aqueous sodium hydroxide (50 ml) and then heated at reflux for 2 hours. The mixture was cooled and evaporated *in vacuo* and the residue was then taken up in ethyl acetate and acidified with 2M aqueous hydrogen chloride. The precipitate was collected by filtration and washed thoroughly with water and then with ether. Drying *in vacuo* gave the title compound (D21) as a yellow solid (2.02 g). [M-H]⁻ = 349.2, RT = 1.82 min

### Description 22

### 1-Methyl-6-(1-methylethyl)-1,6-dihydro[1,2]thiazepino[5,4,3-cd]indole-3,8-dicarboxylic acid 2,2-dioxide (D22)

In a manner analogous to that described for the preparation of compound (D21), dimethyl 1-methyl-6-(1-methylethyl)-1,6-dihydro[1,2]thiazepino[5,4,3-*cd*]indole-3,8-dicarboxylate 2,2-dioxide (D18) was reacted to give the title compound (D22) as a gum. [M-H]⁻ = 363.2, RT = 2.20 min

### Description 23

### 1-Methyl-6-propyl-1,6-dihydro[1,2]thiazepino[5,4,3-cd]indole-3,8-dicarboxylic acid 2,2-dioxide (D23)

In a manner analogous to that described for the preparation of compound (D21), dimethyl 1-methyl-6-propyl-1,6-dihydro[1,2]thiazepino[5,4,3-*cd*]indole-3,8-dicarboxylate 2,2-dioxide (D19) was reacted to give the title compound (D23) as a yellow solid. [M-H]⁻ = 363.2, RT = 2.23 min

### Description 24

### 6-Butyl-1-methyl-1,6-dihydro[1,2]thiazepino[5,4,3-cd]indole-3,8-dicarboxylic acid 2,2-dioxide (D24)

In a manner analogous to that described for the preparation of compound (D21), dimethyl 6-butyl-1-methyl-1,6-dihydro[1,2]thiazepino[5,4,3-*cd*]indole-3,8-dicarboxylate 2,2-dioxide (D20) was reacted to give the title compound (D24) as a fawn solid. [M-H]⁻= 377.2, RT = 2.39 min

### Preparation of Esters

### Ester 1

### Methyl 1,6-diethyl-1,6-dihydro[1,2]thiazepino[5,4,3-cd]indole-8-carboxylate 2,2-dioxide (C1)

A solution of methyl 1-ethyl-1,6-dihydro[1,2]thiazepino[5,4,3-*cd*]indole-8-carboxylate 2,2-dioxide (D11)(0.250 g, 0.82 mmol) in dimethylformamide (15 ml) was treated with a 60% suspension of sodium hydride in oil (0.034 g, 0.85 mmol) under an atmosphere of nitrogen. The mixture was stirred for 10 min and then iodoethane (0.156 g, 1.0 mmol) was added and stirring continued for a further 30 min. A further quantity of first sodium hydride (0.034 g, 0.85 mmol) and then iodoethane (0.156 g, 1.0 mmol) were added and the mixture was left to stand overnight. The solvent was evaporated *in vacuo* and the crude title compound (C1) thus obtained was used in the next step without further purification. [M+H]⁺ = 335.2, RT = 2.83 min

### Ester 2

### Methyl 6-ethyl-1-methyl-1,3,4,6-tetrahydro[1,2]thiazepino[5,4,3-cd]indole-8-carboxylate 2,2-dioxide (C2)

A solution of methyl 1-methyl-1,3,4,6-tetrahydro[1,2]thiazepino[5,4,3*-cd*]indole-8-carboxylate 2,2-dioxide (D13)(0.200 g, 0.68 mmol) in dimethylformamide (15 ml) was treated with a 60% suspension of sodium hydride in oil (0.034 g, 0.85 mmol) under a nitrogen atmosphere and stirred at room temperature for 10 min. The mixture was treated with iodoethane (0.156 g, 1.0 mmol) and stirring was continued for 30 min. The solvent was evaporated *in vacuo* and the residue was dissolved in ethyl acetate and washed sequentially with 2M aqueous hydrogen chloride (50 ml) and saturated aqueous sodium hydrogen carbonate (50 ml). The organic phase was then dried over magnesium sulfate, filtered and evaporated *in vacuo* to yield crude title compound (C2)(0.250 g, 0.78 mmol). This was used without further purification in subsequent reactions. [M+H]⁺ = 323.1, RT = 2.70 min

### Preparation of Acids

### Acid 1

### 6-Ethyl-1-methyl-1,3,4,6-tetrahydro[1,2]thiazepino[5,4,3-cd]indole-8-carboxylic acid 2,2-dioxide (A1)

To a solution of methyl 6-ethyl-1-methyl-1,3,4,6-tetrahydro[1,2]thiazepino[5,4,3-*cd*]indole-8-carboxylate 2,2-dioxide (C2)(0.250 g, 0.78 mmol) in methanol (20 ml) was added 2N aqueous sodium hydroxide solution (10 ml, 20 mmol). The resulting mixture was heated at 50°C until the solution cleared and then the solvent was evaporated *in vacuo.* The residue was extracted with diethyl ether and then the aqueous layer was acidified using 2M aqueous hydrogen chloride and extracted twice with ethyl acetate. The ethyl acetate extracts were dried over MgSO₄, concentrated *in vacuo*, and then triturated with diethyl ether to give the title compound (A1)(0.150 g, 0.49 mmol) as a white solid, which was used in the next step without further purification. [M+H]⁺= 309.1, RT = 2.33 min

### Acid 1 (Alternative Procedure)

### 6-Ethyl-1-methyl-1,3,4,6-tetrahydro[1,2]thiazepino[5,4,3-cd]indole-8-carboxylic acid 2,2-dioxide (A1)

The title compound (A1) may be prepared in an analogous manner to that described for the synthesis of Acid 7 (A7) from 6-ethyl-1-methyl-1,6-dihydro[1,2]thiazepino[5,4,3-*cd*]indole-8-carboxylic acid 2,2-dioxide (A3).

### Acid 2

### 1,6-Diethyl-1,6-dihydro[1,2]thiazepino[5,4,3-cd]indole-8-carboxylic acid 2,2-dioxide (A2)

1,6-Diethyl-1,6-dihydro[1,2]thiazepino[5,4,3-*cd*]indole-8-carboxylic acid 2,2-dioxide (A2) was obtained in an analogous manner to that described for the synthesis of (A1) but using methyl 1,6-diethyl-1,6-dihydro[1,2]thiazepino[5,4,3-*cd*]indole-8-carboxylate 2,2-dioxide (C1) in the place of methyl 6-ethyl-1-methyl-1,3,4,6-tetrahydro[1,2]thiazepino[5,4,3-*cd*]indole-8-carboxylate 2,2-dioxide (C2). [M+H]⁺ = 321.2, RT = 2.45 min

### Acid 3

### 6-Ethyl-1-methyl-1,6-dihydro[1,2]thiazepino[5,4,3-cd]indole-8-carboxylic acid 2,2-dioxide (A3)

6-Ethyl-1-methyl-1,6-dihydro[1,2]thiazepino[5,4,3-*cd*]indole-3,8-dicarboxylic acid 2,2-dioxide (D21) (2.16 g, 6.17mmol) was dissolved in 2N hydrogen chloride in dioxane (120 ml) and heated at reflux for 1 hour. The mixture was then cooled and evaporated *in vacuo* to give a solid. The solid was washed sequentially with water, ether, ethyl acetate, and then with ether again to give the title compound (A3) as a pale yellow solid (1.75 g). [M+H]⁺ = 307.1, RT = 2.18 min

### Acid 4

### 1-Methyl-6-(1-methylethyl)-1,6-dihydro[1,2]thiazepino[5,4,3-cd]indole-8-carboxylic acid 2,2-dioxide (A4)

In an analogous manner to that described for the synthesis of (A3), 1-methyl-6-(1-methylethyl)-1,6-dihydro[1,2]thiazepino[5,4,3-*cd*]indole-3,8-dicarboxylic acid 2,2-dioxide (D22) was reacted to give the title compound (A4) as a pale yellow solid. [M+H]⁺ = 321.2, RT = 2.49 min

### Acid 5

### 1-Methyl-6-propyl-1,6-dihydro[1,2]thiazepino[5,4,3-cd]indole-8-carboxylic acid 2,2-dioxide (A5)

In an analogous manner to that described for the synthesis of Acid 3 (A3), 1-methyl-6-propyl-1,6-dihydro[1,2]thiazepino[5,4,3-*cd*]indole-3,8-dicarboxylic acid 2,2-dioxide (D23) was reacted to give the title compound (A5) as a cream solid. [M+H]⁺ = 321.2, RT = 2.55 min

### Acid 6

### 6-Butyl-1-methyl-1,6-dihydro[1,2]thiazepino[5,4,3-cd]indole-8-carboxylic acid 2,2-dioxide (A6)

In an analogous manner to that described for the synthesis of Acid 3 (A3), 6-butyl-1-methyl-1,6-dihydro[1,2]thiazepino[5,4,3-*cd*]indole-3,8-dicarboxylic acid 2,2-dioxide (D24) was reacted to give the title compound (A6) as a fawn solid. [M+H]⁺ = 355.09, RT = 2.65 min

### Acid 7

### 1-Methyl-6-(1-methylethyl)-1,3,4,6-tetrahydro[1,2]thiazepino[5,4,3-cd]indole-8-carboxylic acid 2,2-dioxide (A7)

A solution of 1-methyl-6-(1-methylethyl)-1,6-dihydro[1,2]thiazepino[5,4,3-*cd*]indole-8-carboxylic acid 2,2-dioxide (A4) (0.22 g, 0.69 mmol) in ethanol:water (9:1, 20 ml) was treated with ammonium formate (0.44 g) and 10% palladium on carbon (0.1 g) and heated at 90°C for 3 hours. On cooling the mixture was filtered and evaporated to dryness *in vacuo*. The residue was dissolved in ethyl acetate and washed sequentially with saturated sodium hydrogen carbonate and water. The organic layer was dried over magnesium sulphate, then filtered and evaporated *in vacuo*. The residue was triturated with ether and the resulting solid was washed with water and then more ether before drying to give the title compound (A7) as a white solid (0.12 g). [M+H]⁺ = 323.2, RT = 2.57 min

### Acid 8

### 1-Methyl-6-propyl-1,3,4,6-tetrahydro[1,2]thiazepino[5,4,3-cd]indole-8-carboxylic acid 2,2-dioxide (A8)

In an analogous manner to that described for the synthesis of Acid 7 (A7), 1-methyl-6-propyl-1,6-dihydro[1,2]thiazepino[5,4,3*-cd*]indole-8-carboxylic acid 2,2-dioxide (A5) was reacted to give the title compound (A8) as a white solid. [M+H]⁺ = 323.2, RT = 2.56 min

### Acid 9

### 6-Butyl-1-methyl-1,3,4,6-tetrahydro[1,2]thiazepino[5,4,3-cd]indole-8-carboxylic acid 2,2-dioxide (A9)

In an analogous manner to that described for the synthesis of Acid (A7), 6-butyl-1-methyl-1,6-dihydro[1,2]thiazepino[5,4,3*-cd*]indole-8-carboxylic acid 2,2-dioxide (A6) was reacted to give the title compound (A9) as a pale yellow solid (0.24 g). [M+H]⁺ = 337.1, RT = 2.75 min

### Acid 10

### 1,6-Diethyl-1,3,4,6-tetrahydro[1,2]thiazepino[5,4,3-cd]indole-8-carboxylic acid 2,2-dioxide (A10)

In an analogous manner to that described for the synthesis of Acid (A7), 1,6-diethyl-1,6-dihydro[1,2]thiazepino[5,4,3*-cd*]indole-8-carboxylic acid 2,2-dioxide (A2) was reacted to give the title compound (A10) as a white solid. [M+H]⁺ = 323.2, RT = 2.50 min

### Preparation of Amines

### Amine 1 (B1)

### (2R,3S)-3-Amino-1-(3-methoxy-benzylamino)-4-phenyl-butan-2-ol di-tosylate

**Step A:** ((S)-(S)-1-Oxiranyl-2-phenyl-ethyl)-carbamic acid *tert*-butyl ester (10 g, 38 mmol) [Chirex 1819W94 Lot#9924382] was dissolved in ethanol (100 ml) and 3-methoxy-benzylamine (14.6 ml, 114 mmol) was added. The resulting mixture was heated, under an atmosphere of nitrogen, for 12 h at reflux temperature. The mixture was cooled and the solvent was removed by evaporation *in vacuo*. The residue was dissolved in ethyl acetate and washed three times with water, dried over magnesium sulfate and concentrated *in vacuo.* Purification by flash chromatography on silica gel (dichloromethane/methanol: 98/2 to 95/5) gave [(1S,2R)-1-benzyl-2-hydroxy-3-(3-methoxy-benzylamino)-propyl]-carbamic acid *tert-*butyl ester (10.0 g, 66%) as a white solid.
**Step B:** To a solution of [(1S,2R)-1-benzyl-2-hydroxy-3-(3-methoxy-benzylamino)-propyl]-carbamic acid *tert*-butyl ester (product of B1, Step A) (10 g, 25 mmol) in acetonitrile (100 ml) was added *p*-toluenesulfonic acid monohydrate (14 g, 75 mmol) and the resulting mixture was stirred for 16 h. The white precipitate formed was filtered and washed with diethyl ether then dried under vacuum to give the title compound (B1) (15.6 g) as a white solid which was used in the next step without further purification.

### Amine 2

### (2R,3S)-3-Amino-4-phenyl-1-(tetrahydro-2H-pyran-4-ylamino)-2-butanol di-tosylate (B2)

**Step A:** ((S)-(S)-1-Oxiranyl-2-phenyl-ethyl)-carbamic acid *tert*-butyl ester (1.1 g, 4.1 mmol) [Chirex 1819W94 Lot#9924382] was dissolved in ethanol (100 ml) and tetrahydro-2*H*-pyran-4-ylamine (0.83 g, 8.22 mmol) was added. The resulting mixture was heated, under an atmosphere of nitrogen, for 4 h at reflux temperature. The mixture was cooled and the solvent was removed by evaporation *in vacuo.* The residue was dissolved in ethyl acetate and washed three times with water, dried over magnesium sulfate and concentrated *in vacuo*. 1,1-Dimethylethyl [(1*S*,2*R*)-2-hydroxy-1-(phenylmethyl)-3-(tetrahydro-2*H*-pyran-4-ylamino)propyl]carbamate was thus obtained as a white solid (0.95 g, 2.6 mmol). [M+H]⁺ = 365.4, RT = 2.16 min
**Step B:** (2*R*,3*S*)-3-Amino-4-phenyl-1-(tetrahydro-2H-pyran-4-ylamino)-2-butanol ditosylate (82) was obtained in an analogous manner to that described for the synthesis of (B1) but using 1,1-dimethylethyl [(1*S*,2*R*)-2-hydroxy-1-(phenylmethyl)-3-(tetrahydro-2*H* pyran-4-ylamino)propyl]carbamate (product of B2, Step A) in the place of [(1S,2R)-1-benzyl-2-hydroxy-3-(3-methoxy-benzylamino)-propyl]-carbamic acid *tert*-butyl ester (product of B1, Step A).

### Amines B3-82

Amines B3-82 were obtained in an analogous manner to that described for Amines 1 and 2 using ((S)-(S)-1-oxiranyl-2-phenyl-ethyl)-carbamic acid *tert*-butyl ester (1.1 g, 4.1 mmol) [Chirex 1819W94 Lot#9924382] and the appropriate amine or an appropriate salt thereof (either obtained from commercial sources or prepared as described in WO 2004/094430). When the salt of the starting amine was used in place of the free base a molar equivalent of an appropriate base (such as triethylamine) was also added to the reaction mixture. The dihydrochloride salt of the amine was prepared in some cases in place of the ditosylate salt. These could be prepared in a manner analogous to that described for Amines (B1) and (B2) but using a solution of 4M HCl in dioxane in the place of p-toluenesulfonic acid monohydrate (as described in WO 2004/094430):

| **Amine No.** | **Name** | **Starting Amine (free base)** |
|---|---|---|
| B3 | (2*R*,3*S*)-3-Amino-4-phenyl-1-({[1-(2,2,2-trifluoroethyl)-1*H*-pyrazol-4- yl]methyl}amino)-2-butanol ditosylate | {[1-(2,2,2-Trifluoroethyl)-1*H*-pyrazol-4-yl]methyl}amine |
| B4 | (2*R*,3*S*)-3-Amino-4-phenyl-1-[(phenylmethyl)amino]-2-butanol ditosylate | Benzylamine |
| B5 | (2*R*,3*S*)-3-Amino-4-phenyl-1-[(4-pyridinylmethyl)amino]-2-butanol ditosylate | (4-Pyridinylmethyl)amine |
| B6 | (2*R*,3*S*)-3-Amino-4-phenyl-1-[(3-pyridinylmethyl)amino]-2-butanol ditosylate | (3-Pyridinylmethyl)amine |
| B7 | (2*R*,3*S*)-3-Amino-1-[(2,2-dimethyltetrahydro-2*H*-pyran-4-yl)amino]-4-phenyl-2-butanol ditosylate | (2,2-Dimethyltetrahydro-2*H*-pyran-4-yl)amine |
| B8 | (2*R*,3*S*)-3-Amino-1-{[(3-ethyl-5-isoxazolyl)methyl]amino}-4-phenyl-2-butanol dihydrochloride | [(3-Ethyl-5-isoxazolyl)methyl]amine |
| B9 | (2*R*,3*S*)-3-Amino-1-(cyclobutylamino)-4-phenyl-2-butanol ditosylate | Cyclobutylamine |
| B10 | (2*R*,3*S*)-3-Amino-1-[(4,4-difluorocyclohexyl)amino]-4-phenyl-2-butanol ditosylate | (4,4-Difluorocyclohexyl)amine |
| B11 | (2*R*,3*S*)-3-Amino-1-[(2-fluoroethyl)amino]-4-phenyl-2-butanol ditosylate | (2-Fluoroethyl)amine |
| B12 | (2*R*,3*S*)-3-Amino-1-[(2,2,3,3,3-pentafluoropropyl)amino]-4-phenyl-2-butanol ditosylate | (2,2,3,3,3-Pentafluoropropyl)amine |
| B13 | (2*R*,3*S*)-3-Amino-1{[(5-ethyl-3-thienyl)methyl]amino}-4-phenyl-2-butanol ditosylate | [(5-Ethyl-3-thienyl)methyl]amine |
| B14 | (2*R*,3*S*)-3-Amino-1-{[2-(methyloxy)ethyl]amino}-4-phenyl-2-butanol ditosylate | [2-(Methyloxy)ethyl]amine |
| B15 | (2*R*,3*S*)-3-Amino-4-phenyl-1-[(2,2,2-trifluoroethyl)amino]-2-butanol ditosylate | (2,2,2-Trifluoroethyl)amine |
| B16 | (2*R*,3*S*)-3-Amino-1-(ethylamino)-4-phenyl-2-butanol ditosylate | Ethylamine |
| B17 | (2*R*,3*S*)-3-Amino-1-[(cyclopropylmethyl)amino]-4-phenyl-2-butanol ditosylate | (Cyclopropylmethyl)amine |
| B18 | (2*R*,3*S*)-3-Amino-1-(cyclohexylamino)-4-phenyl-2-butanol ditosylate | Cyclohexylamine |
| B19 | (2*R*,3*S*)-3-amino-1-(3-cyclopenten-1-ylamino)-4-phenyl-2-butanol ditosylate | 3-Cyclopenten-1-ylamine |
| B20 | (2*R*,3*S*)-3-Amino-1-{[2-(ethylthio)ethyl]amino}-4-phenyl-2-butanol ditosylate | 2-(Ethylthio)ethanamine |
| B21 | (2*R*,3*S*)-3-Amino-1-[(4-methylcyclohexyl)amino]-4-phenyl-2-butanol ditosylate | (4-Methylcyclohexyl)amine |
| B22 | (2*R*,3*S*)-3-Amino-4-phenyl-1-({[3-(trifluoromethyl)phenyl]methyl}amino)-2-butanol ditosylate | {[3-(Trifluoromethyl)phenyl]methyl) amine |
| B23 | (2*R*,3*S*)-3-Amino-4-phenyl-1-[(1-propylbutyl)amino]-2-butanol ditosylate | (1-Propylbutyl)amine |
| B24 | (2*R*,3*S*)-3-Amino-1-[(4,4-dimethylcyclohexyl)amino]-4-phenyl-2-butanol ditosylate | (4,4-Dimethylcyclohexyl)amine |
| B25 | (2*R*,3*S*)-3-Amino-4-phenyl-1-(2-propyn-1-ylamino)-2-butanol ditosylate | 2-Propyn-1-ylamine |
| B26 | (2*R*,3*S*)-3-Amino-4-phenyl-1-(2-propen-1-ylamino)-2-butanol ditosylate | 2-Propen-1-ylamine |
| B27 | (2*R*,3*S*)-3-Amino-1-[(3,3-dimethylbutyl)amino]-4-phenyl-2-butanol ditosylate | (3,3-Dimethylbutyl)amine |
| B28 | (2*R*,3*S*)-3-Amino-4-phenyl-1-[(3,3,5,5-tetramethylcyclohexyl)amino]-2-butanol ditosylate | (3,3,5,5-Tetramethylcyclohexyl)amine |
| B29 | (2*R*,3*S*)-3-Amino-1-[(1,5-dimethylhexyl)amino]-4-phenyl-2-butanol dihydrochloride | (1,5-Dimethylhexyl)amine |
| B30 | (2*R*,3*S*)-3-Amino-4-phenyl-1-(propylamino)-2-butanol dihydrochloride | Propylamine |
| B31 | (2*R*,3*S*)-3-Amino-4-phenyl-1-[(3,3,3-trifluoropropyl)amino]-2-butanol ditosylate | (3,3,3-Trifluoropropyl)amine |
| B32 | (2*R*,3*S*)-3-Amino-1-[(2,2-difluoroethyl)amino]-4-phenyl-2-butanol ditosylate | (2,2-Difluoroethyl)amine |
| B33 | (2*R*,3*S*)-3-Amino-1-[(2-ethylbutyl)amino]-4-phenyl-2-butanol ditosylate | (2-Ethylbutyl)amine |
| B34 | (2*R*,3*S*)-3-Amino-1-[(3-methylbutyl)amino]-4-phenyl-2-butanol ditosylate | (3-Methylbutyl)amine |
| B35 | (2*R*,3*S*)-3-Amino-4-phenyl-1-[(2,2,6,6-tetramethylcyclohexyl)amino]-2-butanol ditosylate | (2,2,6,6-Tetramethylcyclohexyl)amine |
| B36 | (2*R*,3*S*)-3-Amino-1-[(2,2-dimethylcyclohexyl)amino]-4-phenyl-2-butanol ditosylate | (2,2-Dimethylcyclohexyl)amine |
| B37 | (2*R*,3*S*)-3-Amino-1-{[2-(methylthio)ethyl]amino}-4-phenyl-2-butanol ditosylate | [2-(Methylthio)ethyl]amine |
| B38 | (2*R*,3*S*)-3-Amino-1-[(2-cyclohexylethyl)amino]-4-phenyl-2-butanol ditosylate | (2-Cyclohexylethyl)amine |
| B39 | (2*R*,3*S*)-3-Amino-1-[(2-methyl-2-propen-1-yl)amino]-4-phenyl-2-butanol ditosylate | (2-Methyl-2-propen-1-yl)amine |
| B40 | (2*R*,3*S*)-3-Amino-1-(3-buten-1-ylamino)-4-phenyl-2-butanol ditosylate | 3-Buten-1-ylamine |
| B41 | (2*R*,3*S*)-3-Amino-1-(cycloheptylamino)-4-phenyl-2-butanol ditosylate | Cycloheptylamine |
| B42 | (2*R*,3*S*)-3-Amino-4-phenyl-1-(tricyclo[3.3.1.1^{3,7}]dec-2-ylamino)-2-butanol ditosylate | (1*R*,3*S*,5R,7*S*)-Tricyclo[3.3.1.1^{3,7}]dec-2-ylamine |
| B43 | (2*R*,3*S*)-3-Amino-1-[(1*S*,4*R*)-bicycle[2.2.1]hept-2-ylamino]-4-phenyl-2-butanol ditosylate | (1*S*,4*R*)-Bicyclo[2.2.1]hept-2-ylamine |
| 844 | (2*R*,3*S*)-3-Amino-4-phenyl-1-{[2(propyloxy)ethyl]amino}-2-butanol ditosylate | [2-(Propyloxy)ethyl]amine |
| B45 | (2*R*,3*S*)-3-Amino-1-[(1-ethynylcyclohexyl)amino]-4-phenyl-2-butanol ditosylate | (1-Ethynylcyclohexyl)amine |
| B46 | (2*R*,3*S*)-3-Amino-1-[(4-methylphenyl)amino]-4-phenyl-2-butanol ditosylate | 4-Methylaniline |
| B47 | (2*R*,3*S*)-3-Amino-1-[(1methylcyclohexyl)amino]-4-phenyl-2-butanol ditosylate | (1-Methylcyclohexyl)amine |
| B48 | (2*R*,3*S*)-3-Amino-1-[(1-ethylcyclohexyl)amino]-4-phenyl-2-butanol ditosylate | (1-Ethylcyclohexyl)amine |
| B49 | (2*R*,3*S*)-3-Amino-4-phenyl-1-[(1-propylcyclohexyl)amino]-2-butanol ditosylate | (1-Propylcyclohexyl)amine |
| B50 | (2*R*,3*S*)-3-Amino-1-({2-[(1,1-dimethylethyl)thio]ethyl}amino)-4-phenyl-2-butanol ditosylate | {2-[(1,1-Dimethylethyl)thio]ethyl}amine |
| B51 | (2*R*,3*S*)-3-Amino-4-phenyl-1-({2-[(2,2,2-trifluoroethyl)oxy]ethyl)amino)-2-butanol ditosylate | {2-[(2,2,2-Trifluoroethyl)oxy]ethyl}amine |
| B52 | (2*R*,3*S*)-3-Amino-4-phenyl-1-(phenylamino)-2-butanol ditosylate | Aniline |
| B53 | (2*R*,3*S*)-3-Amino-1-[(3-methylphenyl)amino]-4-phenyl-2-butanol ditosylate | 3-Methylaniline |
| B54 | (2*R*,3*S*)-3-Amino-1-[(2-methylphenyl)amino]-4-phenyl-2-butanol ditosylate | 2-Methylaniline |
| B55 | (2*R*,3*S*)-3-Amino-1-{[{1-ethyl-1*H*-pyrazol-4-yl)methyl]amino}-4-phenyl-2-butanol ditosylate | [(1-Ethyl-1*H*-pyrazol-4-yl)methyl]amine |
| B56 | (2*R*,3*S*)-3-Amino-1-[(3-methyl-2-buten-1-yl)amino]-4-phenyl-2-butanol ditosylate | (3-Methyl-2-buten-1-yl)amine |
| B57 | (2*R*,3*S*)-3-Amino-1-[(2-chlorophenyl)amino]-4-phenyl-2-butanol ditosylate | 2-Chloroaniline |
| B58 | (2*R*,3*S*)-3-Amino-1-{[2-(methyloxy)phenyl]amino}-4-phenyl-2-butanol ditosylate | 2-Methoxyaniline |
| B59 | (2*R*,3*S*)-3-Amino-1-{[4-(methyloxy)phenyl]amino}-4-phenyl-2-butanol ditosylate | 4-Methoxyaniline |
| B60 | (2*R*,3*S*)-3-Amino-1-[(3-chlorophenyl)amino]-4-phenyl-2-butanol ditosylate | 3-Chloroaniline |
| B61 | (2*R*,3*S*)-3-Amino-1-{[3-(methyloxy)phenyl]amino}-4-phenyl-2-butanol ditosylate | 3-Methoxyaniline |
| B62 | (2*R*,3*S*)-3-Amino-1-[(4-chlorophenyl)amino]-4-phenyl-2-butanol ditosylate | 4-Chloroaniline |
| B63 | (2*R*,3*S*)-3-Amino-1-(cyclopropylamino)-4-phenyl-2-butanol ditosylate | Cyclopropylamine |
| B64 | (2*R*,3*S*)-3-Amino-1-[(2,4-dimethylphenyl)amino]-4-phenyl-2-butanol ditosylate | 2,4-Dimethylaniline |
| B65 | (2*R*,3*S*)-3-Amino-1-{[4-(dimethylamino)phenyl]amino}-4-phenyl-2-butanol ditosylate | *N*,*N-*Dimethyl-1,4-benzenediamine |
| B66 | (2*R*,3*S*)-3-Amino-1-(2-butyn-1-ylamino)-4-phenyl-2-butanol ditosylate | 2-Butyn-1-ylamine |
| B67 | (2*R*,3*S*)-3-Amino-4-phenyl-1-[(1,1,5-trimethylhexyl)amino]-2-butanol ditosylate | (1,1,5-Trimethylhexyl)amine |
| B68 | (2*R*,3*S*)-3-Amino-1-(butylamino)-4-phenyl-2-butanol ditosylate | Butylamine |
| B69 | (2*R*,3*S*)-3-Amino-1-(cyclooctylamino)-4-phenyl-2-butanol ditosylate | Cyclooctylamine |
| B70 | (2*R*,3*S*)-3-Amino-1-{[2,3-bis(methyloxy)phenyl]amino}-4-phenyl-2-butanol ditosylate | [2,3-Bis(methyloxy)phenyl]amine |
| B71 | (2*R*,3*S*)3-Amino-4-phenyl-1-[({3-[(trifluoromethyl)oxy]phenyl}methyl)amino]-2-butanol ditosylate | ({3-[(Trifluoromethyl)oxy]phenyl)met hyl)amine |
| B72 | (2*R*,3*S*)-3-Amino-1-{[(6-methyl-2-pyridinyl)methyl]amino}-4-phenyl-2-butanol ditosylate | [(6-Methyl-2-pyridinyl)methyl]amine |
| B73 | *N*²-[(2*R*,3*S*)-3-Amino-2-hydroxy-4-phenylbutyl]-*N*¹-cyclohexyl-L-alaninamide dihydrochloride | *N*¹-Cyclohexyl-L-alaninamide |
| B74 | (2*R*,3*S*)-3-Amino-1-{[(1*R*)-1-methylpropyl]amino}-4-phenyl-2-butanol ditosylate | [(1*R*)-1-Methylpropyl]amine |
| B75 | (2*R*,3*S*)-3-Amino-1-{[(*S*)-1-methylpropyl]amino}-4-phenyl-2-butanol ditosylate | [(1*S*)-1-Methylpropyl]amine |
| B76 | (2*R*,3*S*)-3-Amino-4-phenyl-1-[(2-pyridinylmethyl)amino]-2-butanol ditosylate | (2-Pyridinylmethyl)amine |
| B77 | (2*R*,3*S*)-3-Amino-1-{[2-methyl-4-(methyloxy)phenyl]amino}-4-phenyl-2-butanol ditosylate | 2-Methyl-4-(methyloxy)aniline |
| B78 | (2*R*,3*S*)-3-Amino-1-[(1-ethylcyclopropyl)amino]-4-phenyl-2-butanol ditosylate | (1-Ethylcyclopropyl)amine |
| B79 | (2*R*,3*S*)-3-Amino-1-(2-pentyn-1-ylamino)-4-phenyl-2-butanol ditosylate | 2-Pentyn-1-ylamine |
| B80 | (2*R*,3*S*)-3-Amino-1-[(3-fluoropropyl)amino]-4-phenyl-2-butanol ditosylate | (3-Fluoropropyl)amine |
| B81 | (2*R*,3*S*)-3-Amino-1-[(1-methylcyclopropyl)amino]-4-phenyl-2-butanol ditosylate | (1-Methylcyclopropyl)amine |
| B82 | (2*R*,3*S*)-3-Amino-1-[(1,1-dimethyl-2-propyn-1-yl)amino]-4-phenyl-2-butanol ditosylate | (1,1-Dimethyl-2-propyn-1-yl)amine |

### Examples

### Example 1

### 1,6-Diethyl-N-[(1S,2R)-2-hydroxy-1-(phenylmethyl)-3-(tetrahydro-2H-pyran-4-ylamino)propyl]-1,6-dihydro[1,2]thiazepino[5,4,3-cd]indole-8-carboxamide 2,2-dioxide (E1)

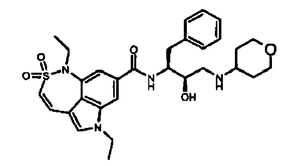

To a solution of 1,6-diethyl-1,6-dihydro[1,2]thiazepino[5,4,3-*cd*]indole-8-carboxylic acid 2,2-dioxide (A2) (0.038 g, 0.12 mmol) in dimethylformamide (3 ml) were added (2*R*,3*S*)-3-amino-4-phenyl-1-(tetrahydro-2*H*-pyran-4-ylamino)-2-butanol di-tosylate (B2)(0.730 g, 0.12 mmol), 1-(3-dimethylaminopropyl)-3-ethyl-carbodiimide hydrochloride (0.030 g, 0.15 mmol), 1-hydroxybenzotriazole hydrate (0.025 g, 0.15 mmol), and triethylamine (0.100 ml, 0.72 mmol). The mixture was stirred overnight at room temperature and then the solvent was evaporated *in vacuo.* The residue was dissolved in ethyl acetate (50 ml) and washed with saturated aqueous sodium hydrogen carbonate (50 ml). The organic phase was dried over magnesium sulfate, filtered and evaporated to give the crude product. Purification by biotage (eluting with 2-5% methanol in dichloromethane) and freeze-drying gave the title compound (E1) (0.030 g, 0.05 mmol) as a white solid.
[M+H]⁺ = 567.6, RT = 2.3 min.

### Examples 2-89 (E2-E89)

Examples 2-89 were obtained in an analogous procedure (in examples where the formate salt is indicated the compounds were purified by mass-directed automated preparative HPLC using acetonitrile/water/formic acid as the eluant rather than by biotage as indicated above) to that described for Example 1 using the appropriate acid and the appropriate amine indicated in the table below:

| **Example** | **Structure** | **Acid** | **Amine** | **[M+H]⁺** | **RT (min)** |
|---|---|---|---|---|---|
| 1,6-Diethyl-*N*-[(1*S*,2*R*)-2-hydroxy-3-({[3-(methyloxy)phenyl]methyl} amino)-1-(phenylmethyl)propyl]-1,6-dihydro[1,2]thiazepino[5,4,3-*cd*]indole-8-carboxamide 2,2-dioxide (Reference E2) | 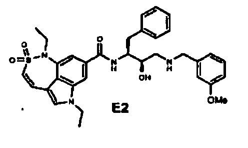 | A2 | B1 | 603.5 | 2.5 |
| 6-Ethyl-*N-*[(1*S*,2*R*)-2-hydroxy-3-({[3-(methyloxy)phenyl]methyl} amino)-1-(phenylmethyl)propyl]-1-methyl-1,3,4,6-tetrahydro[1,2] thiazepino[5,4,3*-cd*]indole-8-carboxamide 2,2-dioxide (E3) | 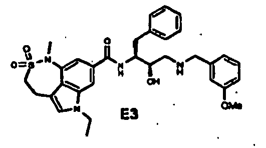 | A1 | B1 | 591.5 | 2.5 |
| 6-Ethyl-*N-*[(1*S*,2*R*)-2-hydroxy-1-(phenylmethyl)-3-(tetrahydro-2*H*-pyran-4-ylamino)propyl]-1-methyl-1,3,4,6-tetrahydro[1,2] thiazepino[5,4,3*-cd*]indole-8-carboxamide 2,2-dioxide (E4) | 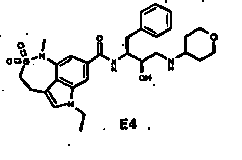 | A1 | B2 | 555.5 | 2.2 |
| Formic acid -6-Ethyl-*N*-[(1*S*,2*R*)-2-hydroxy-1-(phenylmethyl)-3-({[1-(2,2,2-trifluoroethyl)-1*H*-pyrazol-4-yl]methyl}amino)propyl]-1-methyl-1,3,4,6-tetrahydro[1,2]thiazepino[5,4,3*-cd*]indole-8-carboxamide 2,2-dioxide (1:1) (E5) | 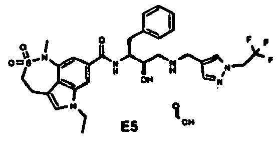 | A1 | B3 | 633.4 | 2.5 |
| Formic acid - 6-Ethyl-*N*-{(1*S*,2*R*)-2-hydroxy-1-(phenylmethyl)-3-[(phenylmethyl)amino]propyl} -1-methyl-1,3,4,6-tetrahydro[1,2]thiazepino[5,4,3*-cd*]indole-8-carboxamide 2,2-dioxide (1:1) (E6) | 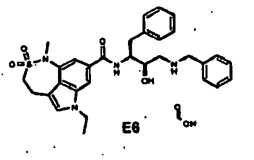 | A1 | B4 | 561.5 | 2.5 |
| Formic acid - 6-Ethyl-*N*-{(1*S*,2*R*)-2-hydroxy-1-(phenylmethyl)-3-[(4-pyridinylmethyl)amino]propyl} -1-methyl-1,3,4,6-tetrahydro[1,2]thiazepino[5,4,3*-cd*]indole-8-carboxamide 2,2-dioxide (1:1) (E7) | 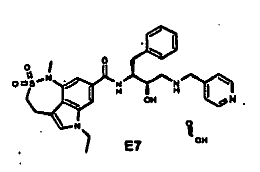 | A1 | B5 | 562.4 | 2.3 |
| Formic acid - 6-Ethyl-*N*-{(1*S*,2*R*)-2-hydroxy-1-(phenylmethyl)-3-[(3-pyridinylmethyl)amino]propyl} -1-methyl-1,3,4,6-tetrahydro[1,2]thiazepino[5,4,3*-cd*]indole-8-carboxamide 2,2-dioxide (1:1) (E8) | 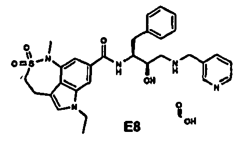 | A1 | B6 | 562.4 | 2.3 |
| Formic acid -*N*-[(1*S*,2*R*)-3-[(2,2-Dimethyltetrahydro-2*H*-pyran-4-yl)amino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E9) | 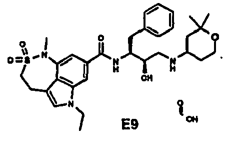 | A1 | B7 | 583.4 | 2.4 |
| 6-Ethyl-*N*-[(1*S*,*2R*)-3-{[(3-ethyl-5-isoxazolyl)methyl]amino}-2-hydroxy-1-(phenylmethyl)propyl]-1-methyl-1,3,4,6-tetrahydro[1,2]thiazepino[5,4,3-*cd*]indole-8-carboxamide 2,2-dioxide (E10) | 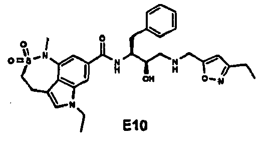 | A1 | B8 | 580.1 | 2.4 |
| Formic acid -*N*-[(1*S*,2*R*)-3-(Cyclobutylamino)-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E11) | 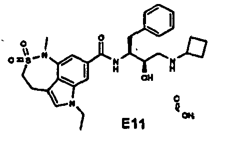 | A1 | B9 | 525.4 | 2.4 |
| Formic acid - *N*-[(1*S*,2*R*)-3-[(4,4-Difluorocyclohexyl) amino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E12) | 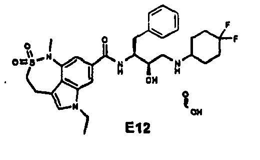 | A1 | B10 | 588.5 | 2.5 |
| Formic acid - 6-Ethyl-*N*-[(1*S*,2*R*)-3-[(2-fluoroethyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide (1:1) (E13) | 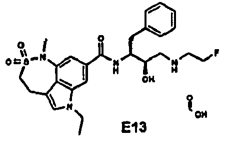 | A1 | B11 | 517.5 | 2.3 |
| 6-Ethyl-*N-*[(1*S*,2*R*)-2-hydroxy-3-[(2,2,3,3,3-pentafluoropropyl)amino]-1-(phenylmethyl)propyl]-1-methyl-1,3,4,6-tetrahydro[1,2]thiazepino[5,4,3-*cd*]indole-8-carboxamide 2,2-dioxide (E14) | 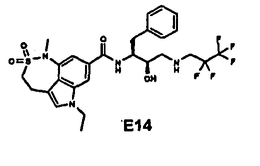 | A1 | B12 | 604.5 | 3.2 |
| Formic acid - 6-Ethyl-*N*-[(1*S*,2*R*)-3-{[(5-ethyl-3-thienyl)methyl]amino}-2-hydroxy-1-(phenylmethyl)propyl]-1-methyl-1,3,4,6-tetrahydro[1,2]thiazepino[5,4,3-*cd*]indole-8-carboxamide 2,2-dioxide (1:1) (E15) | 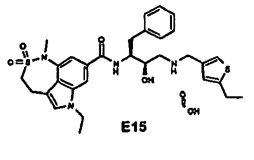 | A1 | B13 | 595.5 | 2.7 |
| Formic acid -6-Ethyl-*N*-[(1*S*,2*R*)-2-hydroxy-3-{[2-(methyloxy)ethyl]amino}-1-(phenylmethyl)propyl]-1-methyl-1,3,4,6-tetrahydro[1,2]thiazepino[5,4,3-*cd*]indole-8-carboxamide 2,2-dioxide (1:1) (E16) | 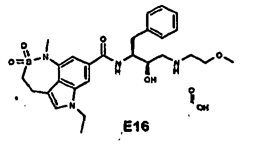 | A1 | B14 | 529.5 | 2.2 |
| 6-Ethyl-*N-*{(1*S*,2*R*)-2-hydroxy-1-(phenylmethyl)-3-[(2,2,2-trifluoroethyl)amino]propyl}-1-methyl-1,3,4,6-tetrahydro[1,2]thiazepino[5,4,3-*cd*]indole-8-carboxamide 2,2-dioxide (E17) | 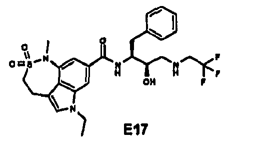 | A1 | B15 | 553.4 | 2.3 |
| Formic acid - 6-Ethyl-*N*-[(1*S*,2*R*)-3-(ethylamino)-2-hydroxy-1-(phenylmethyl)propyl]-1-methyl-1,3,4,6-tetrahydro[1,2]thiazepino[5,4,3*-cd*]indole-8-carboxamide 2,2-dioxide (1:1) (E18) | 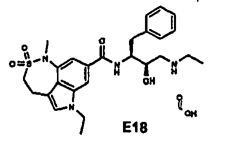 | A1 | B16 | 499.5 | 2.3 |
| Formic acid -*N*-[(1*S*,2*R*)-3-[(Cyclopropylmethyl)amino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E19) | 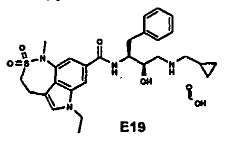 | A1 | B17 | 525.5 | 2.4 |
| Formic acid -*N-*[(1*S*,2*R*)-3-(Cyclohexylamino)-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E20) | 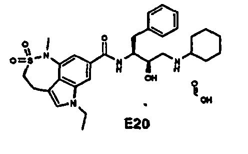 | A1 | B18 | 553.5 | 2.5 |
| Formic acid - *N-*[(1*S*,2*R*)-3-(3-Cyclopenten-1-ylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E21) | 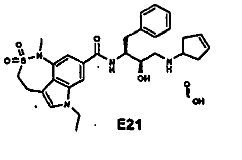 | A1 | B19 | 537.5 | 2.4 |
| Formic acid - 6-Ethyl-*N*-[(1*S*,2*R*)-3-{[2- (ethylthio)ethyl]amino}-2-hydroxy-1-(phenylmethyl)propyl]-1-methyl-1,3,4,6-tetrahydro[1,2]thiazepino[5,4,3*-cd*]indole-8-carboxamide 2,2-dioxide (1:1) (E22) | 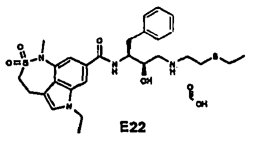 | A1 | B20 | 559.4 | 2.5 |
| Formic acid - 6-Ethyl-*N*-[(1*S*,2*R*)-2-hydroxy-3-[(4-methylcyclohexyl)amino]-1-(phenylmethyl)propyl]-1-methyl-1,3,4,6-tetrahydro[1,2]thiazepino[5,4,3*-cd*]indole-8-carboxamide 2,2-dioxide (1:1) (E23) | 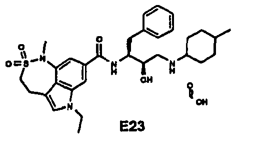 | A1 | B21 | 567.5 | 2.7 |
| Formic acid - 6-Ethyl-*N*-[(1*S*,2*R*)-2-hydroxy-1-(phenylmethyl)-3-({[3-(trifluoromethyl)phenyl]methyl }amino)propyl]-1-methyl-1,3,4,6-tetrahydro[1,2] thiazepino[5,4,3*-cd*]indole-8-carboxamide 2,2-dioxide (1:1) (E24) | 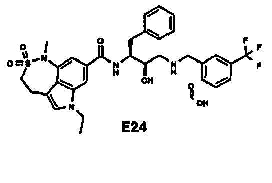 | A1 | B22 | 629.4 | 2.8 |
| Formic acid - 6-Ethyl-*N*-{(1*S*,2*R*)-2-hydroxy-1-(phenylmethyl)-3-[(1-propylbutyl)amino]propyl}-1-methyl-1,3,4,6-tetrahydro[1,2]thiazepino[5,4,3*-cd*]indole-8-carboxamide 2,2-dioxide (1:1) (E25) | 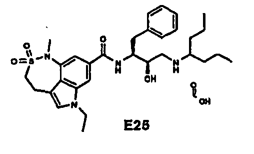 | A1 | B23 | 569.5 | 2.7 |
| Formic acid -*N-*[(1*S*,2*R*)-3-[(4,4-Dimethylcyclohexyl) amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E26) | 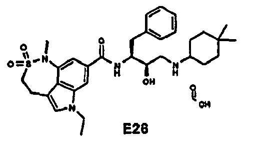 | A1 | B24 | 581.5 | 2.7 |
| 6-Ethyl-*N*-[(1*S*,2*R*)-2-hydroxy-1-(phenylmethyl)-3-(2-propyn-1-ylamino)propyl]-1-methyl-1,3,4,6-tetrahydro[1,2]thiazepino[5,4,3*-cd*]indole-8-carboxamide 2,2-dioxide (E27) | 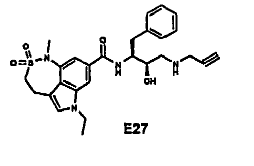 | A1 | B25 | 509.5 | 2.2 |
| Formic acid - 6-Ethyl-*N*-[(1*S*,2*R*)-2-hydroxy-1-(phenylmethyl)-3-(2-propen-1-ylamino)propyl]-1-methyl-1,3,4,6-tetrahydro[1,2] thiazepino[5,4,3*-cd*]indole-8-carboxamide 2,2-dioxide (1:1) (E28) | 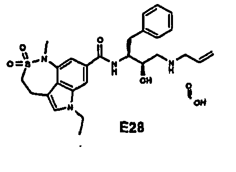 | A1 | B26 | 511.4 | 2.3 |
| Formic acid -*N-*[(1*S*,2*R*)-3-[(3,3-Dimethylbutyl)amino]-2-hydroxy-1-(phenylmethyl) propyl]-6-ethyl-1-methyl-1,3,4,6-tetrahydro[1,2] thiazepino[5,4,3*-cd*]indole-8-carboxamide 2,2-dioxide (1:1) (E29) | 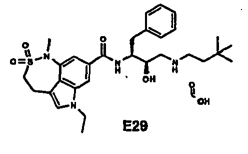 | A1 | B27 | 555.5 | 2.6 |
| Formic acid - 6-Ethyl-*N*-{(1*S*,2*R*)-2-hydroxy-1-(phenylmethyl)-3-[(3,3,5,5-tetramethylcyclohexyl)amino] propyl}-1-methyl-1,3,4,6-tetrahydro[1,2]thiazepino[5,4,3-*cd*]indole-8-carboxamide 2,2-dioxide (1:1) (E30) | 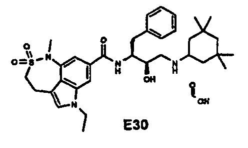 | A1 | B28 | 609.3 | 2.9 |
| *N-*[(1*S*,2*R*)-3-[(1,5-Dimethylhexyl)amino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (E31) | 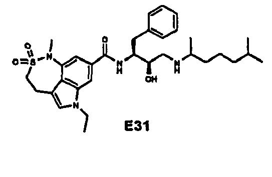 | A1 | B29 | 583.5 | 2.8 |
| 6-Ethyl-*N*-[(1*S*,2*R*)-2-hydroxy-1-(phenylmethyl)-3-(propylamino)propyl]-1-methyl-1,3,4,6-tetrahydro[1,2]thiazepino[5,4,3*-cd*]indole-8-carboxamide 2,2-dioxide (E32) | 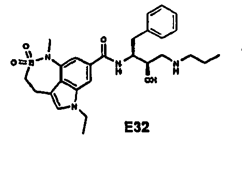 | A1 | B30 | 513.5 | 2.3 |
| 6-Ethyl-*N-*{(1*S*,2*R*)-2-hydroxy-1-(phenylmethyl)-3-[(3,3,3-trifluoropropyl)amino] propyl}-1-methyl-1,3,4,6-tetrahydro[1,2]thiazepino[5,4,3*-cd*]indole-8-carboxamide 2,2-dioxide (E33) | 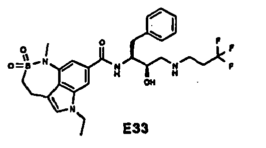 | A1 | B31 | 567.5 | 2.4 |
| *N*-[(1*S*,2*R*)-3-[(2,2-Difluoroethyl)amino]-2-hydroxy-1-(phenylmethyl) propyl]-6-ethyl-1-methyl-1,3,4,6-tetrahydro[1,2] thiazepino[5,4,3*-cd*]indole-8-carboxamide 2,2-dioxide (E34) | 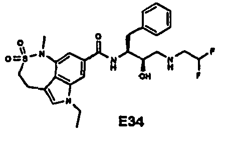 | A1 | B32 | 535.5 | 2.3 |
| Formic acid - 6-Ethyl-*N*-[(1*S*,2*R*)-3-[(2-ethylbutyl)amino]-2-hydroxy-1-(phenylmethyl)propyl]-1-methyl-1,3,4,6-tetrahydro[1,2]thiazepino[5,4,3*-cd*]indole-8-carboxamide 2,2-dioxide (1:1) (E35) | 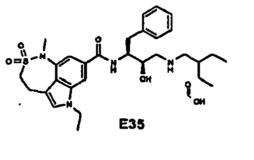 | A1 | B33 | 555.5 | 2.2 |
| Formic acid - 6-Ethyl-*N*-[(1*S*,2*R*)-2-hydroxy-3-[(3-methylbutyl)amino]-1-(phenylmethyl)propyl]-1-methyl-1,3,4,6-tetrahydro[1,2]thiazepino[5,4,3-*cd*]indole-8-carboxamide 2,2-dioxide (1:1) (E36) | 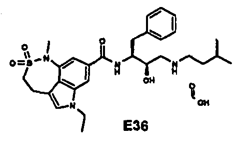 | A1 | B34 | 541.5 | 2.1 |
| Formic acid -6-Ethyl-*N-*{(1*S*,2*R*)-2-hydroxy-1-(phenylmethyl)-3-[(2,2,6,6-tetramethylcyclohexyl)amino] propyl}-1-methyl-1,3,4,6-tetrahydro[1,2]thiazepino[5,4,3*-cd*]indole-8-carboxamide 2,2-dioxide (1:1) (E37) | 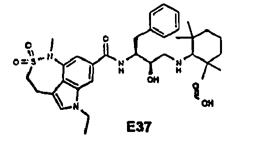 | A1 | B35 | 609.5 | 2.7 |
| Formic acid -*N-*[(1*S*,2*R*)-3-[(2,2-Dimethylcyclohexyl) amino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E38) | 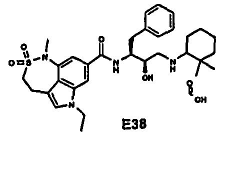 | A1 | B36 | 581.5 | 2.5 |
| 6-Ethyl-*N-*[(1*S*,2*R*)-2-hydroxy-3-{[2-(methylthio)ethyl]amino}-1-(phenylmethyl)propyl]-1-methyl-1,3,4,6-tetrahydro[1,2]thiazepino[5,4,3*-cd*]indole-8-carboxamide 2,2-dioxide (E39) | 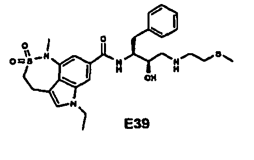 | A1 | B37 | 545.4 | 2.3 |
| Formic acid -*N-*[(1*S*,2*R*)-3-[(2-Cyclohexylethyl)amino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E40) | 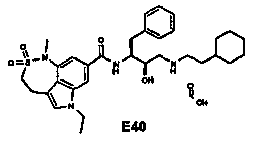 | A1 | B38 | 581.5 | 2.8 |
| Formic acid - 6-Ethyl-*N*-[(1*S*,2*R*)-2-hydroxy-3-[(2-methyl-2-propen-1-yl)amino]-1-(phenylmethyl)propyl]-1-methyl-1,3,4,6-tetrahydro[1,2]thiazepino[5,4,3*-cd*]indole-8-carboxamide 2,2-dioxide (1:1) (E41) | 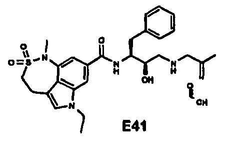 | A1 | B39 | 525.5 | 2.3 |
| Formic acid -*N-*[(1*S*,2*R*)-3-(3-Buten-1-ylamino)-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (1:1) (E42) | 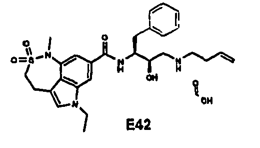 | A1 | B40 | 525.5 | 2.3 |
| *N*-[(1*S*,2*R*)-3-(Cycloheptylamino)-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (E43) | 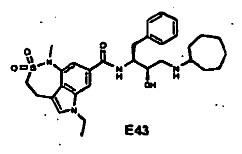 | A1 | B41 | 567.5 | 2.0 |
| 6-Ethyl-*N*-[(1*S*,2*R*)-2-hydroxy-1-(phenylmethyl)-3-(tricyclo[3.3.1.1^{3,7}]dec-2-ylamino)propyl]-1-methyl-1,3,4,6-tetrahydro[1,2]thiazepino[5,4,3*-cd*]indole-8-carboxamide 2,2-dioxide (Reference E44) | 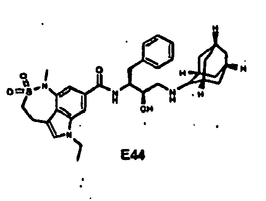 | A1 | B42 | 607.6 | 2.14 |
| *N*-[(1*S*,2*R*)-3-[(1*S*,4*R*)-Bicyclo[2.2.1]hept-2-ylamino]-2-hydroxy-1-(phenylmethyl)propyl]-6-ethyl-1-methyl-1,3,4,6-tetrahydro[1,2]thiazepino[5,4,3*-cd*]indole-8-carboxamide 2,2-dioxide (Reference E45) | 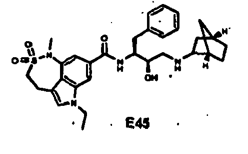 | A1 | B43 | 567.6 | 2.04 |
| Formic acid - 6-Ethyl-*N*-((1*S*,2*R*)-2-hydroxy-1-(phenylmethyl)-3-{[2-(propyloxy)ethyl]amino} propyl)-1-methyl-1,3,4,6-tetrahydro[1,2]thiazepino[5,4,3*-cd*]indole-8-carboxamide 2,2-dioxide (1:1) (E46) | 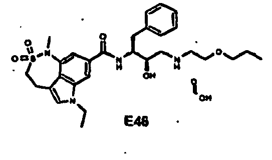 | A1 | B44 | 557.3 | 1.8 |
| Formic acid - 6-Ethyl-*N*-[(1*S*,2*R*)-3-[(1-ethynylcyclohexyl)amino]-2-hydroxy-1-(phenylmethyl)propyl]-1-methyl-1,3,4,6-tetrahydro[1,2]thiazepino[5,4,3*-cd*]indole-8-carboxamide 2,2-dioxide (1:1) (E47) | 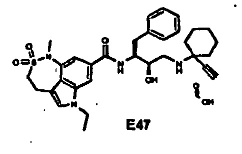 | A1 | B45 | 577.3 | 2.2 |
| 6-Ethyl-*N-*[(1*S*,2*R*)-2-hydroxy-3-[(4-methylphenyl)amino]-1-(phenylmethyl)propyl]-1-methyl-1,3,4,6-tetrahydro[1,2]thiazepino[5,4,3-*cd*]indole-8-carboxamide 2,2-dioxide (E48) | 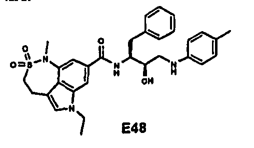 | A1 | B46 | 559.3 | 2.8 |
| Formic acid - 6-Ethyl-*N*-[(1*S*,2*R*)-2-hydroxy-3-[(1-methylcyclohexyl)amino]-1-(phenylmethyl)propyl]-1-methyl-1,3,4,6-tetrahydro[1,2]thiazepino[5,4,3*-cd*]indole-8-carboxamide 2,2-dioxide (1:1) (E49) | 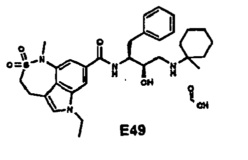 | A1 | B47 | 565.5 | 1.9 |
| Formic acid - 6-Ethyl-*N*-[(1*S*,2*R*)-3-[(1-ethylcyclohexyl)amino]-2-hydroxy-1-(phenylmethyl)propyl]-1-methyl-1,3,4,6-tetrahydro[1,2]thiazepino[5,4,3*-cd*]indole-8-carboxamide 2,2-dioxide (1:1) (E50) | 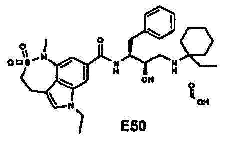 | A1 | B48 | 581.4 | 2.3 |
| Formic acid -6-Ethyl-*N*-{(1*S*,2*R*)-2-hydroxy-1-(phenylmethyl)-3-[(1-propylcyclohexyl)amino] propyl}-1-methyl-1,3,4,6-tetrahydro[1,2]thiazepino[5,4,3-*cd*]indole-8-carboxamide 2,2-dioxide (1:1) (E51) | 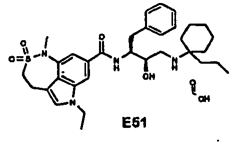 | A1 | B49 | 593.6 | 2.1 |
| Formic acid -*N-*[(1*S*,2*R*)-3-({2-[(1,1-Dimethylethyl)thio]ethyl} amino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E52) | 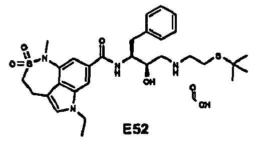 | A1 | B50 | 587.4 | 2.2 |
| Formic acid -6-Ethyl-*N*-[(1*S*,2*R*)-2-hydroxy-1-(phenylmethyl)-3-({2-[(2,2,2-trifluoroethyl)oxy]ethyl}amino) propyl]-1-methyl-1,3,4,6-tetrahydro[1,2]thiazepino[5,4,3*-cd*]indole-8-carboxamide 2,2-dioxide (1:1) (E53) | 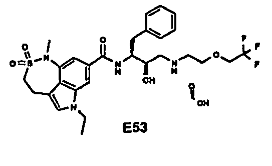 | A1 | B51 | 597.4 | 2.1 |
| Formic acid - 6-Ethyl-*N*-[(1*S*,2*R*)-2-hydroxy-3-(phenylamino)-1-(phenylmethyl)propyl]-1-methyl-1,3,4,6-tetrahydro[1,2]thiazepino[5,4,3*-cd*]indole-8-carboxamide 2,2-dioxide (1:1) (E54) | 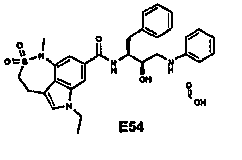 | A1 | B52 | 547.4 | 2.9 |
| Formic acid - 6-Ethyl-*N*-[(1*S*,2*R*)-2-hydroxy-3-[(3-methylphenyl)amino]-1-(phenylmethyl)propyl]-1-methyl-1,3,4,6-tetrahydro[1,2]thiazepino[5,4,3*-cd*]indole-8-carboxamide 2,2-dioxide (1:1) (E55) | 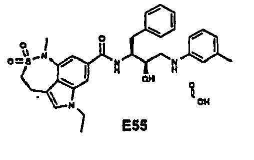 | A1 | B53 | 561.4 | 2.9 |
| Formic acid - 6-Ethyl-*N*-[(1*S*,2*R*)-2-hydroxy-3-[(2-methylphenyl)amino]-1-(phenylmethyl)propyl]-1-methyl-1,3,4,6-tetrahydro[1,2]thiazepino[5,4,3*-cd*]indole-8-carboxamide 2,2-dioxide (1:1) (E56) | 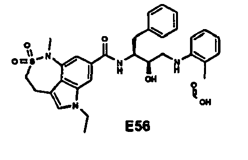 | A1 | B54 | 561.4 | 3.0 |
| Formic acid - 6-Ethyl-*N*-[(1*S*,2*R*)-3-{[(1-ethyl-1*H*-pyrazol-4-yl)methyl]amino}-2-hydroxy-1-(phenylmethyl)propyl]-1-methyl-1, 3,4,6-tetrahydro[1,2]thiazepino[5,4,3*-cd*]indole-8-carboxamide 2,2-dioxide (1:1) (E57) | 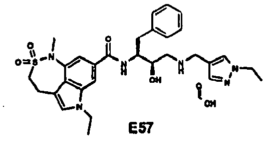 | A1 | B55 | 579.3 | 2.1 |
| Formic acid - 6-Ethyl-*N*-[(1*S*,2*R*)-2-hydroxy-3-[(3-methyl-2-buten-1-yl)amino]-1-(phenylmethyl)propyl]-1-methyl-1,3,4,6-tetrahydro[1,2]thiazepino[5,4,3 *-cd*]indole-8-carboxamide 2,2-dioxide (1:1) (E58) | 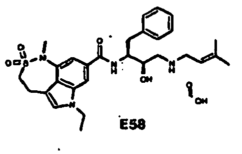 | A1 | B56 | 539.3 | 2.2 |
| Formic acid - 6-Butyl-*N*-[(1*S*,2*R*)-3-(cyclohexylamino)-2-hydroxy-1-(phenylmethyl)propyl]-1-methyl-1,3,4,6-tetrahydro[1,2]thiazepino[5,4,3 *-cd*]indole-8-carboxamide 2,2-dioxide (1:1) (Reference E59) | 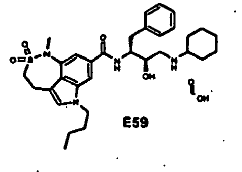 | A9 | B18 | 581.3 | 2.4 |
| Formic acid - *N*-[(1*S*,2*R*)-3-[(2-Chlorophenyl)amino]-2-hydroxy-1-(phenylmethyl)propyl]-6-ethyl-1-methyl-1,3,4,6-tetrahydro[1,2]thiazepino[5,4,3 *-cd*]indole-8-carboxamide 2,2-dioxide (1:1) (E60) | 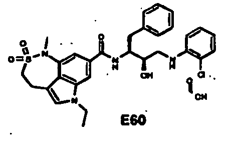 | A1 | B57 | 581.2 | 3.1 |
| Formic acid - 6-Ethyl-*N*-[(1*S*,2*R*)-2-hydroxy-3-{[2-(methyloxy)phenyl]amino}-1-(phenylmethyl)propyl]-1-methyl-1,3,4,6-tetrahydro[1,2]thiazepino[5,4,3 *-cd*]indole-8-carboxamide 2,2-dioxide (1:1) (E61) | 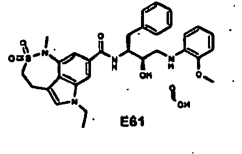 | A1 | B58 | 577.3 | 2.9 |
| Formic acid - 6-Ethyl-*N*-[(1*S*,2*R*)-2-hydroxy-3-{[4-(methyloxy)phenyl]amino}-1-(phenylmethyl)propyl]-1-methyl-1,3,4,6-tetrahydro[1,2]thiazepino[5,4,3-*cd*]indole-8-carboxamide 2,2-dioxide (1:1) (E62) | 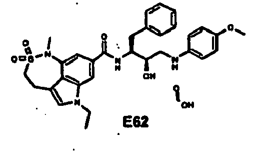 | A1 | B59 | 577.3 | 2.3 |
| Formic acid - *N*-[(1*S*,2*R*)-3-[(3-Chlorophenyl)amino]-2-hydroxy-1-(phenylmethyl)propyl]-6-ethyl-1-methyl-1,3,4,6-tetrahydro[1,2]thiazepino[5,4,3 -*cd*]indole-8-carboxamide 2,2-dioxide (1:1) (E63) | 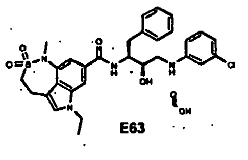 | A1 | B60 | 581.2 | 3.0 |
| Formic acid -6-Ethyl-*N*-[(1*S*,2*R*)-2-hydroxy-3-{[3-(methyloxy)phenyl]amino}-1-(phenylmethyl)propyl]-1-methyl-1,3,4,6-tetrahydro[1,2]thiazepino[5,4,3 -*cd*]indole-8-carboxamide 2,2-dioxide (1:1) (E64) | 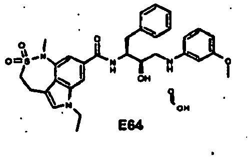 | A1 | B61 | 577.3 | 2.8 |
| Formic acid - *N*-[(1*S*,2*R*)-3-[(4-Chlorophenyl)amino]-2-hydroxy-1-(phenylmethyl)propyl]-6-ethyl-1-methyl-1,3,4,6-tetrahydro[1,2]thiazepino[5,4,3 -*cd*]indole-8-carboxamide 2,2-dioxide (1:1) (E65) | 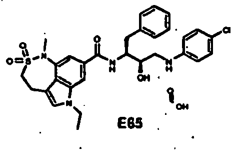 | A1 | B62 | 581.2 | 3.0 |
| Formic acid - *N*-[(1*S*,2*R*)-3-(Cyclohexylamino)-2-hydroxy-1-(phenylmethyl)propyl]-1-methyl-6-propyl-1,3,4,6-tetrahydro[1,2]thiazepino[5,4,3 -*cd*]indole-8-carboxamide 2,2-dioxide (1:1) (Reference E66) | 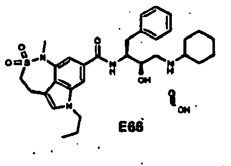 | A8 , | B18 | 567.3 | 2.3 |
| Formic acid - *N*-[(1*S*,2*R*)-3-(Cyclohexylamino)-2-hydroxy-1-(phenylmethyl)propyl]-1-methyl-6-(1-methylethyl)-1,3,4,6-tetrahydro[1,2]thiazepino[5,4,3 -*cd*]indole-8-carboxamide 2,2-dioxide (1:1) (Reference E67) | 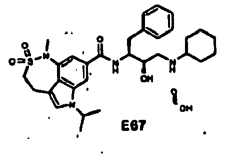 | A7 | B18 | 567.3 | 2.2 |
| *N*-[(1*S*,2*R*)-3-(Cyclopropylamino)-2-hydroxy-1-(phenylmethyl)propyl]-6-ethyl-1-methyl-1,3,4,6-tetrahydro[1,2]thiazepino[5,4,3 -*cd*]indole-8-carboxamide 2,2-dioxide (Reference E68) | 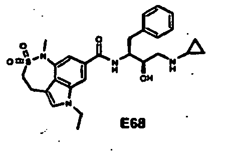 | A1 | 863 | 511.3 | 2.0 |
| Formic acid - *N*-[(1*S*,2*R*)-2-Hydroxy-3-({[3-(methyloxy)phenyl]methyl} amino)-1-(phenylmethyl)propyl]-1-methyl-6-propyl-1,3,4,6-tetrahydro[1,2]thiazepino[5,4,3 -*cd*]indole-8-carboxamide 2,2-dioxide (1:1) (Reference E69) | 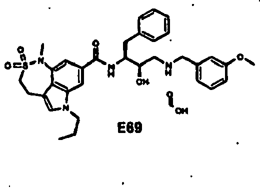 | A8 | B1 | 605.4 | 2.2 |
| Formic acid - *N*-[(1*S*,2*R*)-3-(Cyclohexylamino)-2-hydroxy-1-(phenylmethyl)propyl]-1,6-diethyl-1,3,4,6-tetrahydro[1,2]thiazepino[5,4,3 -*cd*]indole-8-carboxamide 2,2-dioxide (1:1) (E70) | 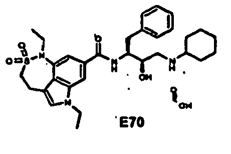 | A10 | B18 | 567.4 | 2.1 |
| Formic acid - *N*-[(1*S*,2*R*)-3-[(2,4-Dimethylphenyl)amino]-2-hydroxy-1-(phenylmethyl)propyl]-6-ethyl-1-methyl-1,3,4,6-tetrahydro[1,2]thiazepino[5,4,3 -*cd*]indole-8-carboxamide 2,2-dioxide (1:1) (E71) | 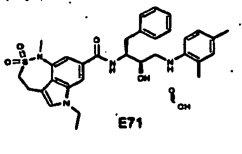 | A1 | B64 | 575.4 | 2.9 |
| Formic acid - *N*-[(1*S*,2*R*)-3-{[4-(Dimethylamino)phenyl] amino}-2-hydroxy-1-(phenylmethyl)propyl]-6-ethyl-1-methyl-1,3,4,6-tetrahydro[1,2]thiazepino[5,4,3 -*cd*]indole-8-carboxamide 2,2-dioxide (1:1) (E72) | 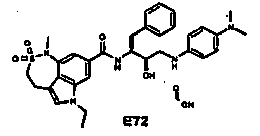 | A1 | B65 | 590.4 | 2.1 |
| Formic acid - *N*-[(1*S*,2*R*)-3-(2-Butyn-1-ylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E73) | 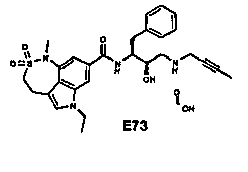 | A1 | B66 | 523.3 | 2.1 |
| Formic acid - 6-Ethyl-*N*-{(1*S*,2*R*)-2-hydroxy-1-(phenylmethyl)-3-[(1,1,5-trimethylhexyl)amino]propyl}-1-methyl-1,3,4,6-tetrahydro[1,2]thiazepino[5,4,3-*cd*]indole-8-carboxamide 2,2-dioxide (1:1) (E74) | 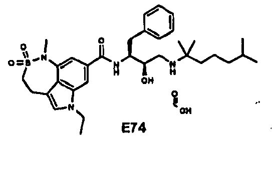 | A1 | B67 | 597.5 | 2.4 |
| Formic acid -*N*-[(1*S*,2*R*)-3-(Butylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E75) | 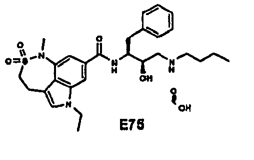 | A1 | B68 | 527.4 | 2.1 |
| Formic acid - *N*-[(1*S*,2*R*)-3-{[2,3-Bis(methyloxy)phenyl] amino}-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E76) | 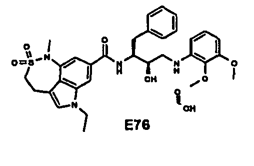 | A1 | B70 | 607.4 | 2.9 |
| Formic acid - 6-Ethyl-*N*-{(1*S*,2*R*)-2-hydroxy-1-(phenylmethyl)-3-[({3-[(trifluoromethyl)oxy]phenyl} methyl)amino]propyl}-1-methyl-1,3,4,6-tetrahydro[1,2]thiazepino[5,4,3-*cd*]indole-8-carboxamide 2,2-dioxide (1:1) (E77) | 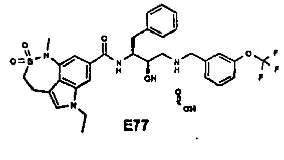 | A1 | B71 | 645.4 | 2.3 |
| Formic acid - 6-Ethyl-*N*-[(1*S*,2*R*)-2-hydroxy-3-{[(6-methyl-2-pyridinyl)methyl]amino}-1-(phenylmethyl)propyl]-1-methyl-1,3,4,6-tetrahydro[1,2]thiazepino[5,4,3-*cd*]indole-8-carboxamide 2,2-dioxide (1:1) (E78) | 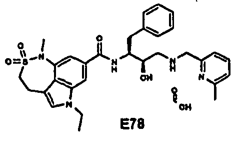 | A1 | B72 | 576.4 | 2.2 |
| Formic acid - *N*-[(1*S*,2*R*)-3-{[(1*S*)-2-(Cyclohexylamino)-1-methyl-2-oxoethyl]amino}-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E79) | 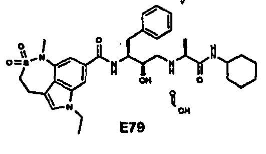 | A1 | B73 | 624.5 | 2.2 |
| Formic acid - 6-Ethyl-*N*-[(1*S*,2*R*)-2-hydroxy-3-{[(1*R*)-1-methylpropyl]amino}-1-(phenylmethyl)propyl]-1-methyl-1,3,4,6-tetrahydro[1,2]thiazepino[5,4,3-*cd*]indole-8-carboxamide 2,2-dioxide (1:1) (E80) | 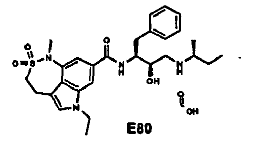 | A1 | B74 | 527.4 | 2.1 |
| Formic acid - 6-Ethyl-*N*-[(1*S*,2*R*)-2-hydroxy-3-{[(1*S*)-1-methylpropyl]amino}-1-(phenylmethyl)propyl]-1-methyl-1,3,4,6-tetrahydro[1,2]thiazepino[5,4,3-*cd*]indole-8-carboxamide 2,2-dioxide (1:1) (E81) | 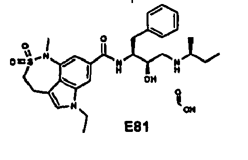 | A1 | B75 | 527.4 | 2.1 |
| Formic acid - 6-Ethyl-*N*-{(1*S*,2*R*)-2-hydroxy-1-(phenylmethyl)-3-[(2-pyridinylmethyl)amino]propyl} -1-methyl-1,3,4,6-tetrahydro[1,2]thiazepino[5,4,3-*cd*]indole-8-carboxamide 2,2-dioxide (1:1) (E82) | 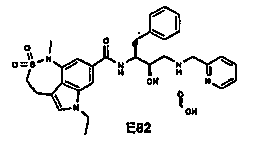 | A1 | B76 | 562.3 | 2.1 |
| Formic acid - 6-Ethyl-*N*-[(1*S*, 2*R*)-2-hydroxy-3-{[2-methyl-4-(methyloxy)phenyl]amino}-1-(phenylmethyl)propyl]-1-methyl-1,3,4,6-tetrahydro[1,2]thiazepino[5,4,3-*cd*]indole-8-carboxamide 2,2-dioxide (1:1) (E83) | 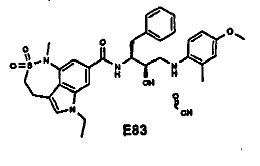 | A1 | B77 | 591.2 | 2.2 |
| Formic acid - 6-Ethyl-*N*-[(1*S*, 2*R*)-3-[(1-ethylcyclopropyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide (1:1) (E84) | 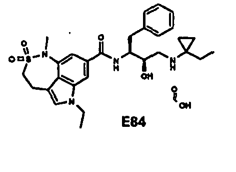 | A1 | B78 | 539.3 | 2.1 |
| Formic acid - 6-Ethyl-*N* [(1*S*,2*R*)-2-hydroxy-3-(2-pentyn-1-ylamino)-1-(phenylmethyl)propyl]-1-methyl-1,3,4,6-tetrahydro[1,2]thiazepino[5,4,3-*cd*]indole-8-carboxamide 2,2-dioxide (1:1) (E85) | 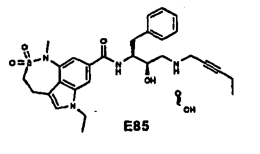 | A1 | B79 | 537.3 | 2.1 |
| Formic acid - 6-Ethyl-*N*-[(1*S*,2*R*)-3-[(3-fluoropropyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide (1:1) (E86) | 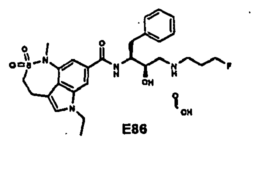 | A1 | B80 | 531.3 | 2.0 |
| Formic acid - 6-Ethyl-*N*-[(1*S*,2*R*)-2-hydroxy-3-[(1-methylcyclopropyl)amino]-1-(phenylmethyl)propyl]-1-methyl-1,3,4,6-tetrahydro[1,2]thiazepino[5,4,3-*cd*]indole-8-carboxamide 2,2-dioxide (1:1) (E87) | 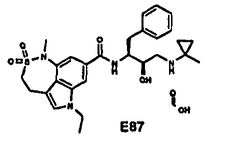 | A1 | B81 | 525.4 | 2.1 |
| Formic acid - *N*-[(1*S*,2*R*)-3-[(1,1-Dimethyl-2-propyn-1-yl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (1:1) (E88) | 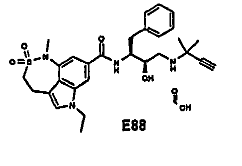 | A1 | B82 | 537.6 | 1.9 |
| *N*-[(1*S*,2*R*)-3-(Cyclooctylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide (E89) | 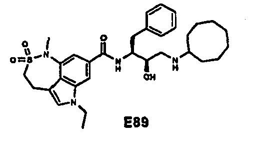 | A1 | B69 | 581.5 | 2.1 |

### Example 90

### 1,6-Diethyl-N-[(1S,2R)-2-hydroxy-1-(phenylmethyl)-3-(tetrahydro-2H-pyran-4-ylamino)propyl]-1,3,4,6-tetrahydro[1,2]thiazepino[5,4,3-cd]indole-8-carboxamide 2,2-dioxide (E90)

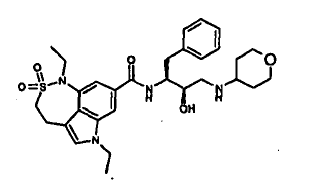

A solution of 1,6-diethyl-*N*-[(1*S*,2*R*)-2-hydroxy-1-(phenylmethyl)-3-(tetrahydro-2*H*-pyran-4-ylamino)propyl]-1,6-dihydro[1,2]thiazepino[5,4,3-*cd*]indole-8-carboxamide 2,2-dioxide (E1) (0.010 g, 0.02 mmol) in methanol (5 ml) was treated with ammonium formate (0.020 g, 0.32 mmol) and 10% palladium on charcoal (0.015 g) and heated at reflux for 1h. The mixture was filtered and evaporated *in vacuo*. The residue was dissolved in ethyl acetate (50 ml) and washed with saturated aqueous sodium hydrogen carbonate (30 ml). The organic phase was dried over magnesium sulfate, filtered and evaporated *in vacuo.* Freeze-drying gave the title compound (E90) (0.005 g, 0.01 mmol) as a white solid. [M+H]⁺ = 569.6, RT = 2.3 min.

Compounds of the invention may be tested for *in vitro* biological activity in accordance with the following assays:

### (I) Asp-2 inhibitory assay

For each compound being assayed, in a 384 well plate, is added:-
a) 1µl of a DMSO solution of the test compound (IC₅₀ curve uses ten 1 in 2 serial dilutions from 500 µM).
b) 10 µl of substrate (FAM-SEVNLDAEFK-TAMRA) solution in buffer. This is prepared by diluting 2ml of a 2mM DMSO solution of the substrate into 400ml of buffer (100mM Sodium acetate pH = 4.5, 1 I Milli-Q water, 0.06% Triton X-100 (0.5 ml/l), pH adjusted to 4.5 using glacial acetic acid). Aminomethyl fluorescein (FAM) and tetramethyl rhodamine (TAMRA) are fluorescent molecules which co-operate to emit fluorescence at 535nm upon cleavage of the SEVNLDAEFK peptide.
c) 10 µl enzyme solution. This is prepared by diluting 16ml of a 500nM enzyme solution into 384 ml of buffer (prepared as above).
   Blank wells (enzyme solution replaced by buffer) are included as controls on each plate. Wells are incubated for 1 h at room temperature and fluorescence read using a Tecan Ultra Fluorimeter/Spectrophotometer (485nm excitation, 535nm emission).

### (II) Cathepsin D inhibitory assay

For each compound being assayed, in a 384 well plate, is added:-
a) 1µl of a DMSO solution of the test compound (IC₅₀ curve uses ten 1 in 2 serial dilutions from 500 µM).
b) 10 µl of substrate (FAM-SEVNLDAEFK-TAMRA) solution in buffer. This is prepared by diluting 2ml of a 2mM DMSO solution of the substrate into 400ml of buffer (100mM Sodium acetate pH = 4.5, 1 I Milli-Q water, 0.06% Triton X-100 (0.5 ml/l), pH adjusted to 4.5 using glacial acetic acid).
c) 10 µl enzyme solution. This is prepared by diluting 1.6ml of a 200 unit/ml (in 10 mM HCl) enzyme solution into 398.4 ml of buffer (prepared as above).
   Blank wells (enzyme solution replaced by buffer) are included as controls on each plate. Wells are incubated for 1 h at room temperature and fluorescence read using a Tecan Ultra Fluorimeter/Spectrophotometer (485nm excitation, 535nm emission).

### Pharmacological Data

The compounds of E1-E90 were tested in Assays (I) and (II) and exhibited inhibition within the following range: 2nM - 10µM (Asp2) and 30nM - >100µM (catD).

### Abbreviations

- DMF: dimethylformamide
- DMSO: dimethylsulfoxide
- DMAP: dimethylaminophenol
- DABCO: 1,4-diazabicyclo [2.2.2] octane
- DME: dimethyl ether
- THF: tetrahydrofuran
- HOBT: N-hydroxybenzotriazole
- [ ]: single amino acid letter code relating to peptide sequence

## Claims

1. A compound of formula (I): 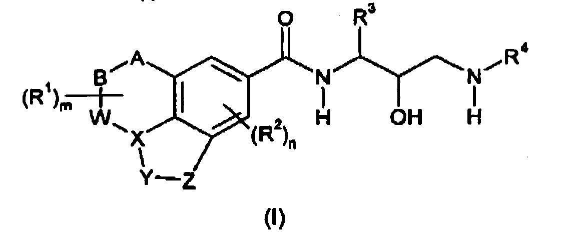 wherein
m represents 0;
n represents 0;
A-B represents -NR⁵-SO₂-;
R⁵ represents methyl or ethyl;
-W- represents -(CH₂)₂-;
X-Y-Z represents -C=CR⁸-NR⁹-;
R⁸ represents hydrogen;
R⁹ represents ethyl;
R³ represents unsubstituted benzyl;
R⁴ represents
- C₁₋₁₀ alkyl optionally substituted by one or more halogen, C₁₋₆ alkoxy, haloC₁₋₆ alkoxy or -S-C₁₋₆ alkyl groups;
- C₂₋₁₀ alkenyl optionally substituted by one or more C₁₋₆ alkyl groups;
- C₃₋₁₀ alkynyl optionally substituted by one or more C₁₋₆ alkyl groups;
- C₃₋₁₀ cycloalkyl optionally substituted by one or more halogen, C₁₋₆ alkyl or -C₂₋₆ alkynyl groups;
- C₃₋₁₀ cycloalkenyl;
- C₁₋₆ alkyl-C₃₋₁₀ cycloalkyl;
- benzyl or phenyl, which benzyl or phenyl is optionally substituted by one or more halogen, cyano, haloC₁₋₆ alkoxy, haloC₁₋₆ alkyl, C₁₋₆ alkyl, C₁-₈ alkoxy or -N(Me)₂ groups;
- CH₂-pyrazolyl, - CH₂-pyridinyl, - CH₂-thienyl or - CH₂-isoxazolyl, which -CH₂-pyrazolyl, -CH₂-pyridinyl, -CH₂-thienyl or -CH₂-isoxazolyl is optionally substituted by one or more halogen, cyano, haloC₁₋₆ alkoxy, haloC₁₋₆ alkyl, C₁₋₆ alkyl or C₁₋₆ alkoxy groups;
- C(R^{a}R^{b})-CONH-C₃₋₁₀ cycloalkyl ; or
- tetrahydropyranyl optionally substituted by one or more C₁₋₆ alkyl groups;
R^{a} and R^{b} independently represent hydrogen, methyl or together with the carbon atom to which they are attached form a cyclopropyl or cyclohexyl group;
or a pharmaceutically acceptable salt or solvate thereof.

2. A compound according to claims 1, wherein R⁴ represents -C₁₋₁₀ alkyl optionally substituted by one or more halogen, C₁₋₆ alkoxy, haloC₁₋₆ alkoxy or -S-C₁₋₆ alkyl groups.

3. A compound according to claim 1 which is:
6-Ethyl-*N*-[(1*S*,2*R*)-2-hydroxy-3-({[3-(methyloxy)phenyl]methyl} amino)-1-(phenylmethyl)propyl]-1-methyl-1,3,4,6-tetrahydro[1,2] thiazepino[5,4,3-*cd*]indole-8-carboxamide 2,2-dioxide;
6-Ethyl-*N*-[(1*S*,2*R*)-2-hydroxy-1-(phenylmethyl)-3-(tetrahydro-2*H*-pyran-4-ylamino)propyl]-1-methyl-1,3,4,6-tetrahydro[1,2] thiazepino[5,4,3-*cd*]indole-8-carboxamide 2,2-dioxide;
6-Ethyl-*N*-[(1*S*,2*R*)-2-hydroxy-1-(phenylmethyl)-3-({[1-(2,2,2-trifluoroethyl)-1*H*-pyrozol-4-yl]methyl}amino)propyl]-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(phenylmethyl)amino]propyl}-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(4-pyridinylmethyl)amino]propyl]-1-methyl-1,3,4,6-tetrahydro[1,2]thiazopino[5,4,3-*cd*]indole-8-carboxamide 2,2-dioxide;
6-Ethyl-*N*-{(1*S*,2*R*)-2-hydroxy-1-(phenylmethyl)-3-[(3-pyridinylmethyl)amino]propyl}-1-methyl-1,3,4,6-tetrahydro[1,2]thiazepino[5,4,3-*cd*]indole-8-carboxamide 2,2-dioxide;
*N*-[(1*S*,2*R*)-3-1(2,2-Dimethyltetrahydro-2*H*-pyran-4-yl)amino]-2-hydroxy-1-(phenylmethyl)propyl]-6-ethyl-1-methyl-1,3,4,6-tetrahydro[1,2]thiazepino[5,4,3-*cd*] indole-8-carboxamide 2,2-dioxide;
6-Ethyl-*N*-[(1*S*,2*R*)-3-{[(3-ethyl-5-isoxazolyl)methyl]amino}-2-hydroxy-1-(phenylmethyl)propyl]-methyl-1,3,4,6-tetrahydro[1,2]thiazepino[5,4,3-*cd*]indole-8-carboxamide 2,2-dioxide;
*N*-{(1*S*,2*R*)-3-(Cyclobutylamino)-2-hydroxy-1-(phenylmethyl) propyl]-6-ethyl-1-methyl-1,3,4,6-tetrahydro[1,2]thiazepino[5,4,3-*cd*]indole-8-carboxamide 2,2-dioxide;
*N*-[(1*S*,2*R*)-3-[(4,4-Difluorocyclohexyl) amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*, 2*R*)-3-[(2-fluoroethyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(2,2,3,3,3-pentafluoropropyl)amino]-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-{[(5-ethyl-3-thienyl)methyl]amino}-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[2-(methyloxy)ethyl]amino}-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(2,2,2-trifluoroethyl)amino]propyl}-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-(ethylamino)-2-hydroxy-1-(phenylmethyl)propyl]-1-methyl-1,3,4,6-tetrahydro[1,2]thiazepino[5,4,3-*cd*]indole-8-carboxamide 2,2-dioxide;
*N*-[(1*S*,2*R*)-3-[(Cyclopropylmethyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
*N*-[(1*S*,2*R*)-3-(Cyclohexylamino)-2-hydroxy-1-(phenylmethyl)propyl}-6-ethyl-1-methyl-1,3,4,6-tetrahydro[1,2]thiazopino[5,4,3-*cd*]indole-8-carboxamide 2,2-dioxide;
*N*-[(1*S*,2*R*)-3-(3-Cyclopenten-1-ylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-{[2-(ethylthio)ethyl]amino}-2-hydroxy-1-(phenylmethyl) propyl]-1-methyl-1,3,4,6-tetrahydro[1,2]thiazepino[5,4,3-*cd*]indole-8-carboxamide 2,2-dioxide;
8-Ethyl-*N*-[(1*S*,2*R*)-2-hydroxy-3-[(4-methylcyclohexyl)amino]-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-1-(phenylmethyl)-3-({[3-(trifluoromethyl)phenyl]methyl}amino)propyl]-1-methyl-1,3,4,6-tetrahydro[1,2] thiazepino[5,4,3-*cd*]indole-8-carboxamide 2,2-dioxide;
6-Ethyl-*N*-{(1*S*,2*R*)-2-hydroxy-1-(phenylmethyl)-3-[(1-propylbutyl)amino]propyl}-1-methyl-1,3,4,6-tetrahydro[1,2]thiazepino[5,4,3-*cd*]indole-8-carboxamide 2,2-dioxide;
*N*-[(1*S*,2*R*)-3-[(4,4-Dimethylcyclohexyl) amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-1-(phenylmethyl)-3-(2-propyn-1-ylamino)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-1-(phenylmethyl)-3-(2-propen-1-ylamino)propyl]-1-methyl-1,3,4,6-tetrahydro[1,2] thiazepino[5,4,3-*cd*]indole-8-carboxamide 2,2-dioxide;
*N*-[(1*S*,2*R*)-3-[(3,3-Dimethylbutyl)amino]-2-hydroxy-1-(phenylmethyl) propyl]-6-ethyl-1-methyl-1,3,4,6-tetrahydro[1,2] thiazepino[5,4,3-*cd*]indole-8-carboxamide 2,2-dioxide;
6-Ethyl-*N*-{(1*S*,2*R*)-2-hydroxy-1-(phenylmethyl)-3-[(3,3,5,5-tetramethylcyclohexyl)amino]propyl}-1-methyl-1,3,4,6-tetrahydro[1,2]thiazepino[5,4,3-*cd*]indole-8-carboxamide 2,2-dioxide;
*N*-[(1*S*,2*R*)-3-[(1,5-Dimethylhexyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-1-(phenylmethyl)-3-(propylamino)propyl]-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(3,3,3-trifluoropropyl)amino] propyl}-1-methyl-1,3,4,8-tetrahydro[1,2]thiazepino[5,4,3-*cd*]indole-8-carboxamide 2,2-dioxide;
*N*-[(1S,2R)-3-[(2,2-Difluoroethyl)amino]-2-hydroxy-1-(phenylmethyl) propyl]-6-ethyl-1-methyl-1,3,4,6-tetrahydro[1,2] thiazepino[5,4,3-*cd*]indole-8-carboxamide 2,2-dioxide;
6-Ethyl-*N*-[(1*S*,2*R*)-3-[(2-ethylbutyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(3-methylbutyl)amino]-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(2,2,6,6-tetramethylcyclohexyl)amino]propyl}-1-methyl-1,3,4,8-tetrahydro[1,2]thiazepino[5,4,3-*cd*]indole-6-carboxamide 2,2-dioxide;
*N*-[(1*S*,2*R*)-3-[(2,2-Dimethylcyclohexyl) amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide:
6-Ethyl-*N*-[(1*S*,2*R*)-2-hydroxy-3-{[2-(methylthio)ethyl]amino}-1-(phenylmethyl)propyl]-1-methyl-1,3,4,6-tetrahydro[1,2]thiazepino[5,4,3-*cd*]indole-8-carboxamide 2,2-dioxide;
*N*-[(1*S*,2*R*)-3-[(2-Cyclohexylethyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(2-methyl-2-propen-1-yl)amino]-1-(phenylmethyl)propyl]-1-methyl-1,3,4,6-tetrahydro[1,2]thiazepino[5,4,3-*cd*]indole-8-carboxamide 2,2-dioxide;
*N*-[(1*S*,2*R*)-3-(3-Buten-1-ylamino)-2-hydroxy-1-(phenylmethyl)propyl]-6-ethyl-1-methyl-1,3,4,8-tetrahydro[1,2]thiazepino[5,4,3-*cd*]indole-8-carboxamide 2,2-dioxide;
*N*-[(1*S*,2*R*)-3-(Cycloheptylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-((1*S*,2*R*)-2-hydroxy-1-(phenylmethyl)-3-{[2-(propyloxy)ethyl]amino} propyl)-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-[(1-ethynylcyclohexyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(4-methylphenyl)amino]-1-(phenylmethyl)propyl]-1-methyl-1,3,4,6-tetrahydro[1,2]thiazepino[5,4,3-*cd*]indole-8-carboxamide 2,2-dioxide;
8-Ethyl-*N*-[(1*S*,2*R*)-2-hydroxy-3-[(1-methylcyclohexyl)amino]-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-[(1-ethylcyclohexyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(1-propylcyclohexyl)amino] propyl}-1-methyl-1,3,4,6-tetrahydro[1,2]thiazapino[5,4,3-*cd*]indole-8-carboxamide 2,2-dioxide;
*N*-[(1*S*,2*R*)-3-({2-[(1,1-Dimethylethyl)thio]ethyl}amino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-1-(phenylmethyl)-3-({2-[(2,2,2-trifluoroethyl)oxy]ethyl}amino)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-(phenylamino)-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(3-methylphenyl)amino]-1-(phenylmethyl)propyl]-1-methyl-1,3,4,8-tetrahydro[1,2]thiazepino[5,4,3-*cd*]indole-8-carboxamide 2,2-dioxide;
6-Ethyl-*N*-[(1*S*,2*R*)-2-hydroxy-3-[(2-methylphenyl)amino]-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-{[(1-ethyl-1H-pyrazol-4-yl)methyl]amino}-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(3-methyl-2-buten-1-yl)amino]-1-(phenylmethyl) propyl}. 1-methyl-1,3,4,6-tetrahydro[1,2]thiazepino[5,4,3-*cd*]indole-8-carboxamide 2,2-dioxide;
*N*-[(1*S*,2*R*)-3-[(2-Chlorophenyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[2-(methyloxy)phenyl]amino)1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[4-(methyloxy)phenyl]amino}-1-(phenylmethyl)propyl]-1-methyl-1,3,4,6-tetrahydro[1,2]thiazepino[5,4,3-*cd*]indole-8-carboxamide 2,2-dioxide;
*N*-[(1*S*,2*R*)-3-[(3-Chlorophenyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[3-(methyloxy)phenyl]amino}-1-(phenylmethyl)propyl]-1-methyl-1,3,4,6-tetrahydro[1,2]thiazepino[5,4,3-*cd*]indole-8-carboxamide 2,2-dioxide;
*N*-[(1*S*,2*R*)-3-[(4-Chlorophenyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
*N*-[(1*S*,2*R*)-3-(Cyclopropylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
*N*-[(1*S*,2*R*)-3-(Cyclohexylamino)-2-hydroxy-1-(phenylmethyl)propyl]-1,6-diethyl-1,3,4,6-tetrahydro[1,2]thiazepino[5,4,3-*cd*]indole-8-carboxamide 2,2-dioxide;
*N*-[(1*S*,2*R*)-3-[(2,4-Dimethylphenyl)amino]-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
*N***-**[(1*S*,2*R*)-3-{[4-(Dimethylamino)phenyl]amino}-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
*N*-[(1*S*,2*R*)-3-(2-Butyn-1-ylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(1,1,5-trimethylhexyl)amino]propyl}-1-methyl-1,3,4,6-tetrahydro[1,2]thiazepino[5,4,3-*cd*]indole-8-carboxamide 2,2-dioxice;
*N*-[(1*S*,2*R*)-3-(Butylamino)-2-hydroxy-1-(phenylmethyl)propyl]-8-ethyl-1-methyl-1,8,4,6-tetrahydro[1,2]thiazepino[5,4,3-*cd*]indole-8-carboxamide 2,2-dioxide;
*N*-[(1*S*,2*R*)-3-{[12,3-Bis(methyloxy)phenyl]amino}-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[({3-[(trifluoromethyl)oxy]phenyl}methyl)amino]propyl}-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[(6-methyl-2-pyridinyl)methyl]amino}-1-(phenylmethyl)propyl]-1-methyl-1,3,4,6-tetrahydro[1,2]thiazepino[5,4,3-*cd*]indole-8-carboxamide 2,2-dioxide:
*N*-[(1*S*,2*R*)-3-{[(1*S*)-2-(Cyclohexylamino)-1-methyl-2-oxoethyl]amino}-2-hydroxy-1-(phenylmethyl)propyl]-6-ethyl-1-methyl-1,3,4,8-tetrahydro[1,2]thiazepino[5,4,3-*cd*]indole-8-carboxamide 2,2-dioxide;
6-Ethyl-*N*-[(1*S*,2*R*)-2-hydroxy-3-{[(1*R*)-1-methylpropyl]amino}-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[(1*S*)-1-methylpropyl]amino}-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-{(1*S*,2*R*)-2-hydroxy-1-(phenylmethyl)-3-[(2-pyridinylmethyl)amino]propyl}-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-{[2-methyl-4-(methyloxy)phenyl]amino}-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3-[(1-ethylcyclopropyl)amino]-2-hydroxy-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-(2-pentyn-1-ylamino)-1-(phenylmethyl)propyl]-1-methyl-1,3,4,6-tetrahydro[1,2]thiazepino[5,4,3-*cd*]indole-8-carboxamide 2,2-dioxide;
6-Ethyl-*N*-[(1*S*,2*R*)-3[(3-fluoropropyl)amino]-2-hydroxy-1-(phenylmethyl) propyl]-1. methyl-1,3,4,6-tetrahydro[1,2]thiazepino[5,4,3-*cd*]indole-8-carboxamide 2,2-dioxide;
6-Ethyl-*N*-[(1*S*,2*R*)-2-hydroxy-3-[(1-methylcyclopropyl)amino]-1-(phenylmethyl)propyl]-1-methyl-1,3,4,6-tetrahydro[1,2]thiazepino[5,4,3-*cd*]indole-8-carboxamide 2,2-dioxide;
*N*-[(1*S*,2*R*)-3-[(1,1-Dimethyl-2-propyn-1-yl)amino]-2-hydroxy-1-(phenylmethyl)propyl]-6 ethyl-1-methyl-1,3,4,6-tetrahydro[1,2]thiazepino[5,4,3-*cd*]indole-8-carboxamide 2,2-dioxide;
*N*-[(1*S*,2*R*)-3-(Cyclooctylamino)-2-hydroxy-1-(phenylmethyl)propyl]-6-ethyl-1-methyl-1,3,4,6-tetrahydro[1,2]thiazepino[5,4,3-*cd*]indole-8-carboxamide 2,2-dioxide;
1,6-Diethyl-*N*-[(1*S*,2*R*)-2-hydroxy-1-(phenylmethyl)-3-(tetrahydro-2H-pyran-4-ylamino)propyl]1,3,4,6-tetrahydro[1,2]thiazepino[5,4,3-*cd*]indole-8-carboxamide 2,2-dioxide;
or a pharmaceutically acceptable salt or solvate thereof.

4. A pharmaceutical composition comprising a compound of formula (I) as defined in any preceding claim or a pharmaceutically acceptable salt or solvate thereof in admixture with one or more pharmaceutically acceptable diluents or carriers.

5. A compound of formula (I) as defined in any one of claims 1 to 3 or a pharmaceutically acceptable salt or solvate thereof for use as a pharmaceutical.

6. Use of a compound of formula (I) as defined in any one of claims 1 to 3 or a pharmaceutically acceptable salt or solvate thereof in the manufacture of a medicament for the treatment of diseases **characterised by** elevated β-amyloid levels or β-amyloid deposits.

## Patentansprüche

1. Verbindung der Formel (I): 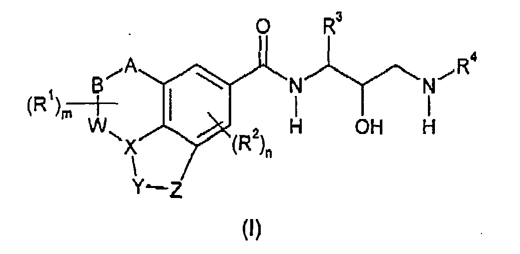 wobei
m für 0 steht;
n für 0 steht;
A-B für -NR⁵-SO₂- steht;
R⁵ für Methyl oder Ethyl steht;
-W- für -(CH₂)₂- steht;
X-Y-Z für -C=CR⁸-NR⁹ steht;
R⁸ Wasserstoff darstellt;
R⁹ Ethyl darstellt;
R³ unsubstituiertes Benzyl darstellt;
R⁴ für folgende steht
- C₁-₁₀-Alkyl, gegebenenfalls substituiert mit einem oder mehreren Halogenatomen, C₁-₆-Alkoxy-, Halogen-C₁₋₆-alkoxy- oder -S-C₁₋₆-Alkylresten;
- C₂₋₁₀-Alkenyl, gegebenenfalls substituiert mit einem oder mehreren C₁₋₆-Alkylresten;
- C₃₋₁₀-Alkinyl, gegebenenfalls substituiert mit einem oder mehreren C₁₋₆-Alkylresten;
- C₃₋₁₀-Cycloalkyl, gegebenenfalls substituiert mit einem oder mehreren Halogenatomen, C₁₋₆-Alkyl oder -C₂₋₆-Alkinylresten;
- C₃₋₁₀-Cycloalkenyl;
- C₁₋₆-Alkyl-C₃₋₁₀-cycloalkyl;
- Benzyl oder Phenyl, wobei Benzyl oder Phenyl gegebenenfalls substituiert sind mit einem oder mehreren Halogenatomen, Cyanogruppen, Halogen-C₁₋₆-alkoxy-, Halogen-C₁₋₆-alkyl-, C₁₋₆-Alkyl-, C₁₋₆-Alkoxy- oder -N(Me)₂-Resten;
- CH₂-Pyrazolyl, -CH₂-Pyridinyl, -CH₂-Thienyl oder -CH₂-Isoxazolyl, wobei -CH₂-Pyrazolyl, -CH₂-Pyridinyl, -CH₂-Thienyl oder -CH₂-Isoxazolyl gegebenenfalls substituiert sind mit einem oder mehreren Halogenatomen, Cyanogruppen, Halogen-C ₁₋₆-alkoxy-, Halogen-C₁₋₆-alkyl-, C₁₋₆-Alkyl- oder C₁₋₆-Alkoxyresten;
- C(R^{a}R^{b})-CONH-C₃₋₁₀-Cycloalkyl; oder
- Tetrahydropyranyl, gegebenenfalls substituiert mit einem oder mehreren C₁₋₆-Alkylresten;
R^{a} und R^{b} unabhängig Wasserstoff oder Methyl darstellen oder zusammen mit dem Kohlenstoffatom, an das sie gebunden sind, einen Cyclopropyl- oder Cyclohexylrest bilden;
oder ein pharmazeutisch verträgliches Salz oder Solvat davon.

2. Verbindung nach Anspruch 1, wobei R⁴ für -C₁₋₁₀-Alkyl steht, gegebenenfalls substituiert mit einem oder mehreren Halogenatomen, C₁₋₆-Alkoxy-, Halogen-C₁₋₆-Alkoxy- oder -S-C₁₋₆-Alkylresten.

3. Verbindung nach Anspruch 1, nämlich:
6-Ethyl-N-[(1S,2R)-2-hydroxy-3-({[3-(methyloxy)phenyl]methyl} amino)-1 - (phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-1-(phenylmethyl)-3-(tetrahydro-2H-pyran-4-ylamino)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-1-(phenylmethyl)-3-({[1-(2,2,2-trifluorethyl)-1H-pyrazol-4-yl]methyl}amino)propyl]-1-methyl-1,3,4,6-tetrahydro[1,2]thiazepino[5,4,3-*cd*]indol-8-carboxamid-2,2-dioxid;
6-Ethyl-N-{(1S,2R)-2-hydroxy-1-(phenylmethyl)-3-[(phenylmethyl)amino]propyl}-1-methyl-1,3,4,6-tetrahydro[1,2]thiazepino[5,4,3-*cd*]indol-8-carboxamid-2,2-dioxid;
6-Ethyl-N-{(1S,2R)-2-hydroxy-1-(phenylmethyl)-3-[(4-pyridinylmethyl)amino]propyl}-1-methyl-1,3,4,6-tetrahydro[1,2]thiazepino[5,4,3-*cd*]indol-8-carboxamid-2,2-dioxid;
6-Ethyl-N- {(1S,2R)-2-hydroxy-1-(phenylmethyl)-3-[(3-pyridinylmethyl)amino]propyl}-1-methyl-1,3,4,6-tetrahydro[1,2] thiazepino[5,4,3-*cd*]indol-8-carboxamid-2,2-dioxid;
N-[(1S,2R)-3-[(2,2-Dimethyltetrahydro-2H-pyran-4-yl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-3-{[(3-ethyl-5-isoxazolyl)methyl]amino}-2-hydroxy-1-(phenylmethyl)propyl]-1-methyl-1,3,4,6-tetrahydro[1,2]thiazepino[5,4,3-*cd*]indol-8-carboxamid-2,2-dioxid;
N-[(1S,2R)-3-(Cyclobutylamino)-2-hydroxy-1-(phenylmethyl)propyl]-6-ethyl-1-methyl-1,3,4,6-tetrahydro [1,2]thiazepino[5,4,3-*cd*]indol-8-carboxamid-2,2-dioxid;
N-[(1S,2R)-3-[(4,4-Difluorcyclohexyl)amino]-2-hydroxy-1-(phenylmethyl)propyl]-6-ethyl-1-methyl-1,3,4,6-tetrahydro[ 1,2]thiazepino[5,4,3-*cd*]indol-8-carboxamid-2,2-dioxid;
6-Ethyl-N-[(1S,2R)-3-[(2-fluorethyl)amino]-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(2,2,3,3,3-pentafluorpropyl)amino]-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-3-{[(5-ethyl-3-thienyl)methyl]amino}-2-hydroxy-1-(phenylmethyl)propyl]-1-methyl-1,3,4,6-tetrahydro[1,2] thiazepino [5,4,3-*cd*]indol-8-carboxamid-2,2-dioxid;
6-Ethyl-N-[(1S,2R)-2-hydroxy-3-{[2-(methyloxy)ethyl]amino} -1-(phenylmethyl)propyl]-1-methyl-1,3,4,6-tetrahydro[1,2]thiazepino[5,4,3-*cd*]indol-8-carboxamid-2,2-dioxid;
6-Ethyl-N- {(1S,2R)-2-hydroxy-1-(phenylmethyl)-3-[(2,2,2-trifluorethyl)amino]propyl}-1-methyl-1,3,4,6-tetrahydro[1,2]thiazepino[5,4,3-*cd*]indol-8-carboxamid-2,2-dioxid;
6-Ethyl-N-[(1S,2R)-3-(ethylamino)-2-hydroxy-1-(phenylmethyl)propyl]-1-methyl-1,3,4,6-tetrahydro[1,2]thiazepino[5,4,3-*cd*]indol-8-carboxamid-2,2-dioxid;
N-[(1S,2R)-3-[(Cyclopropylmethyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-(Cyclohexylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-(3-Cyclopenten-1-ylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-3-{[2-(ethylthio)ethyl]amino-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(4-methylcyclohexyl)amino]-1-(phenylmethyl)propyl]-1-methyl-1,3,4,6-tetrahydro[ 1,2]thiazepino[5,4,3-*cd*]indol-8-carboxamid-2,2-dioxid;
6-Ethyl-N-[(1S,2R)-2-hydroxy-1-(phenylmethyl)-3-({[3-(trifluormethyl)phenyl]methyl}amino)propyl]-1-methyl-1,3,4,6-tetrahydro[ 1,2]thiazepino[5,4,3-*cd*]indol-8-carboxamid-2,2-dioxid;
6-Ethyl-N-{(1S,2R)-2-hydroxy-1-(phenylmethyl)-3-[(1-propylbutyl)amino]propyl}-1-methyl-1,3,4,6-tetrahydro[1,2]thiazepino[5,4,3-*cd*]indol-8-carboxamid-2,2-dioxid;
N-[(1S,2R)-3-[(4,4-Dimethylcyclohexyl)amino]-2-hydroxy-1-(phenylmethyl)propyl]-6-ethyl-1-methyl-1,3,4,6-tetrahydro [1,2]thiazepino[5,4,3-*cd*]indol-8-carboxamid-2,2-dioxid;
6-Ethyl-N-[(1S,2R)-2-hydroxy-1-(phenylmethyl)-3-(2-propin-1-ylamino)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-1-(phenylmethyl)-3-(2-propen-1-ylamino)propyl]-1-methyl-1,3,4,6-tetrahydro[1,2]thiazepino[5,4,3-*cd*]indol-8-carboxamid-2,2-dioxid;
N-[(1S,2R)-3-[(3,3-Dimethylbutyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N- {(1S,2R)-2-hydroxy-1-(phenylmethyl)-3-[(3,3,5,5-tetramethylcyclohexyl)amino]propyl}-1-methyl-1,3,4,6-tetrahydro[1,2]thiazepino[5,4,3-*cd*]indol-8-carboxamid-2,2-dioxid;
N-[(1S,2R)-3-[(1,5-Dimethylhexyl)amino]-2-hydroxy-1-(phenylmethyl)propyl]-6-ethyl-1-methyl-1,3,4,6-tetrahydro[ 1,2]thiazepino[5,4,3-*cd*]indol-8-carboxamid-2,2-dioxid;
6-Ethyl-N-[(1S,2R)-2-hydroxy-1-(phenylmethyl)-3-(propylamino)propyl]-1-methyl-1,3,4,6-tetrahydro[1,2]thiazepino[5,4,3-*cd*]indol-8-carboxamid-2,2-dioxid;
6-Ethyl-N-{(1S,2R)-2-hydroxy-1-(phenylmethyl)-3-[(3,3,3-trifluorpropyl)amino]propyl}-1-methyl-1,3,4,6-tetrahydro[1,2]thiazepino[5,4,3-*cd*]indol-8-carboxamid-2,2-dioxid;
N-[(1S,2R)-3-[(2,2-Difluorethyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid; 6-Ethyl-N-[(1S,2R)-3-[(2-ethylbutyl)amino]-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(3-methylbutyl)amino]-1-(phenylmethyl)propyl]-1-methyl-1,3,4,6-tetrahydro[1,2]thiazepino[5,4,3-*cd*]indol-8-carboxamid-2,2-dioxid; 6-Ethyl-N- {(1S,2R)-2-hydroxy-1-(phenylmethyl)-3-[(2,2,6,6-tetramethylcylohexyl)amino]propyl)-1-methyl-1,3,4,6-tetrahydro[1,2] thiazepino [5,4,3-*cd*]indol-8-carboxamid-2,2-dioxid;
N-[(1S,2R)-3-[(2,2-Dimethylcyclohexyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{[2-(methylthio)ethyl]amino}-1-(phenylmethyl)propyl]-1-methyl-1,3,4,6-tetrahydro[1,2]thiazepino[5,4,3-*cd*]indol-8-carboxamid-2,2-dioxid;
N-[1S,2R)-3-[(2-Cyclohexylethyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(2-methyl-2-propen-1-yl)amino]-1-(phenylmethyl)propyl]-1-methyl-1,3,4,6-tetrahydro[1,2]thiazepino[5,4,3-*cd*]indol-8-carboxamid-2,2-dioxid;
N-[(1S,2R)-3-(3-Buten-1-ylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-(Cycloheptylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-((1S,2R)-2-hydroxy-1-(phenylmethyl)-3-{[2-(propyloxy)ethyl]amino }propyl)-1-methyl-1,3,4,6-tetrahydro[1,2]thiazepino[5,4,3-*cd*]indol-8-carboxamid-2,2-dioxid;
6-Ethyl-N-[(1S,2R)-3-[(1-ethinylcyclohexyl)amino]-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(4-methylphenyl)amino]-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(1-methylcyclohexyl)amino]-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-3-[(1-ethylcyclohexyl)amino]-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N- {(1S,2R)-2-hydroxy-1-(phenylmethyl)-3-[(1-propylcyclohexyl)amino]propyl}-1-methyl-1,3,4,6-tetrahydro[1,2]thiazepino[5,4,3-*cd*]indol-8-carboxamid-2,2-dioxid;
N-[(1S,2R)-3-({2-[(1,1-Dimethylethyl)thio]ethyl}amino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-1-(phenylmethyl)-3-({ 2-[(2,2,2-trifluorethyl)oxy]ethyl}amino)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-(phenylamino)-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(3-methylphenyl)amino]-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(2-methylphenyl)amino]-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-3-{[(1-ethyl-1H-pyrazol-4-yl)methyl]amino)-2-hydroxy-1 - (phenylmethyl)propyl]-1-methyl-1,3,4,6-tetrahydro[1,2]thiazepino[5,4,3-cd]indol-8-carboxamid-2,2-dioxid;
6-Ethyl-N-[(1S,2R)-2-hydroxy-3-[(3-methyl-2-buten-1-yl)amino]-1-(phenylmethyl)propyl]-1-methyl-1,3,4,6-tetrahydro[1,2]thiazepino[5,4,3-*cd*]indol-8-carboxamid-2,2-dioxid;
N-[(1*S*,2*R*)-3-[(2-Chlorphenyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{[2-(methyloxy)phenyl]amino}-1-(phenylmethyl)propyl]-1-methyl-1,3,4,6-tetrahydro-[1,2]thiazepino[5,4,3-*cd*]indol-8-carboxamid-2,2-dioxid;
6-Ethyl-N-[(1S,2R)-2-hydroxy-3-{[4-(methyloxy)phenyl]amino}-1-(phenylmethyl)-propyl]-1-methyl-1,3,4,6-tetrahydro[1,2]thiazepino[5,4,3-*cd*]indol-8-carboxamid-2,2-dioxid;
N-[(1S,2R)-3-[(3-Chlorphenyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{[3-(methyloxy)phenyl]amino}-1-(phenylmethyl)propyl]-1-methyl-1,3,4,6-tetrahydro[1,2]thiazepino[5,4,3-cd]indol-8-carboxamid-2,2-dioxid;
N-[(1S,2R)-3-[(4-Chlorphenyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-(Cyclopropylamino)-2-hydroxy-1-(phenylmethyl)propyl]-6-ethyl-1 - methyl-1,3,4,6-tetrahydro[1,2]thiazepino [5,4,3-*cd*]indol-8-carboxamid-2,2-dioxid;
N-[(1S,2R)-3-(Cyclohexylamino)-2-hydroxy-1-(phenylmethyl)propyl]-1,6-diethyl-1,3,4,6-tetrahydro[1,2]thiazepino[5,4,3-cd]indol-8-carboxamid-2,2-dioxid;
N-[(1S,2R)-3-[(2,4-Dimethylphenyl)amino]-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-{[4-(Dimethylamino)phenyl]amino}-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-(2-Butin-1-ylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-{(1S,2R)-2-hydroxy-1-(phenylmethyl)-3-[(1,1,5-trimethylhexyl)amino]propyl}-1-methyl-1,3,4,6-tetrahydro[1,2]thiazepino[5,4,3-cd]indol-8-carboxamid-2,2-dioxid;
N-[(1S,2R)-3-(Butylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
N-[(1S,2R)-3-{[2,3-Bis(methyloxy)phenyl]amino}-2-hydroxy-1-(phenylmethyl)propyl]-6-ethyl-1-methyl-1,3,4,6-tetrahydro[1,2]thiazepino[5,4,3-cd]indol-8-carboxamid-2,2-dioxid;
6-Ethyl-N-{(1S,2R)-2-hydroxy-1-(phenylmethyl)-3-[({3-[(trifluormethyl)oxy]phenyl}methyl)amino]propyl}-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{[(6-methyl-2-pyridinyl)methyl]amino}-1-(phenylmethyl)propyl]-1-methyl-1,3,4,6-tetrahydro[1,2]thiazepino[5,4,3-*cd*]indol-8-carboxamid-2,2-dioxid;
N-[(1S,2R)-3-{[(1S)-2-(Cylohexylamino)-1-methyl-2-oxoethyl]amino}-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{[(1R)-1-methylpropyl]amino}-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{[(1S)-1-methylpropyl]amino}-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-{(1S,2R)-2-hydroxy-1-(phenylmethyl)-3-[(2-pyridinylmethyl)amino]propyl} - 1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-{ [2-methyl-4-(methyloxy)phenyl]amino}-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-3-[(1-ethylcyclopropyl)amino]-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-(2-pentin-1-ylamino)-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-3-[(3-fluorpropyl)amino]-2-hydroxy-1-(phenylmethyl)propyl]-1-methyl-1,3,4,6-tetrahydro[1,2]thiazepino[5,4,3-*cd*]indol-8-carboxamid-2,2-dioxid;
6-Ethyl-N-[(1S,2R)-2-hydroxy-3-[(1-methylcyclopropyl)amino]-1-(phenylmethyl)propyl]-1-methyl-1,3,4,6-tetrahydro[1,2]thiazepino[5,4,3-cd]indol-8-carboxamid-2,2-dioxid;
N-[(1S,2R)-3-[(1,1-Dimethyl-2-propin-1-yl)amino]-2-hydroxy-1-(phenylmethyl)-propyl]-6-ethyl-1-methyl-1,3,4,6-tetrahydro[1,2]thiazepino[5,4,3-*cd*]indol-8-carboxamid-2,2-dioxid;
N-[(1S,2R)-3-(Cyclooctylamino)-2-hydroxy-1-(phenylmethyl)propyl]-6-ethyl-1-methyl-1,3,4,6-tetrahydro[1,2]thiazepino[5,4,3-*cd*]indol-8-carboxamid-2,2-dioxid;
1,6-Diethyl-N-[(1S,2R)-2-hydroxy-1-(phenylmethyl)-3-(tetrahydro-2H-pyran-4-ylamino)propyl]-1,3,4,6-tetrahydro[1,2]thiazepino[5,4,3-*cd*]indol-8-carboxamid-2,2-dioxid;
oder ein pharmazeutisch verträgliches Salz oder Solvat davon.

4. Arzneimittel umfassend eine Verbindung der Formel (I) wie in einem der vorstehenden Ansprüche definiert oder ein pharmazeutisch verträgliches Salz oder Solvat davon in Beimischung mit einem oder mehreren pharmazeutisch verträglichen Verdünnungsmitteln oder Trägern.

5. Verbindung der Formel (I) wie in einem der Ansprüche 1 bis 3 definiert oder ein pharmazeutisch verträgliches Salz oder Solvat davon zur Verwendung als ein Arzneimittel.

6. Verwendung einer Verbindung der Formel (I) wie in einem der Ansprüche 1 bis 3 definiert oder eines pharmazeutisch verträglichen Salzes oder Solvats davon bei der Herstellung eines Medikaments zur Behandlung von Krankheiten, die durch erhöhte β-Amyloidgehalte oder β-Amyloidablagerungen **gekennzeichnet** sind.

## Revendications

1. Composé de formule (I) : 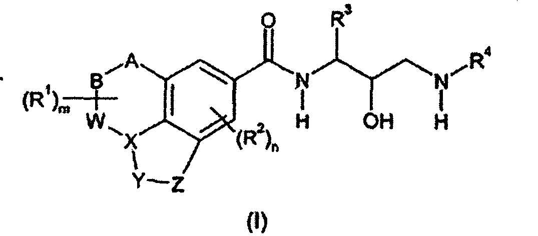 dans laquelle
m est égal à 0 ;
n est égal à 0 ;
A-B représente un groupe -NR⁵-SO₂-;
R⁵ représente un groupe méthyle ou éthyle ;
-W- représente un groupe -(CH₂)₂- ;
X-Y-Z représente un groupe -C=CR⁸-NR⁹- ;
R⁸ représente un atome d'hydrogène ;
R⁹ représente un groupe éthyle ;
R³ représente un groupe benzyle non substitué ;
R⁴ représente un groupe
- alkyle en C₁ à C₁₀ facultativement substitué avec un ou plusieurs groupes halogéno, alkoxy en C₁ à C₆, halogénalkoxy en C₁ à C₆ ou -S-alkyle en C₁ à C₆ ;
- alcényle en C₂ à C₁₀ facultativement substitué avec un ou plusieurs groupes alkyle en C₁ à C₆ ;
- alcynyle en C₃ à C₁₀ facultativement substitué avec un ou plusieurs groupes alkyle en C₁ à C₆ ;
- cycloalkyle en C₃ à C₁₀ facultativement substitué avec un ou plusieurs groupes halogéno, alkyle en C₁ à C₆ ou
- alcynyle en C₂ à C₆ ;
- cycloalcényle en C₃ à C₁₀
- (alkyle en C₁ à C₆) - (cycloalkyle en C₃ à C₁₀);
- benzyle ou phényle, groupe benzyle ou phényle qui est facultativement substitué avec un ou plusieurs groupes halogéno, cyano, halogénalkoxy en C₁ à C₆, halogénalkyle en C₁ à C₆, alkyle en C₁ à C₆ , alkoxy en C₁ à C₆ ou -N(Me)₂ ;
- CH₂-pyrazolyle, -CH₂-pyridinyle, -CH₂-thiényle ou -CH₂-isoxazolyle, groupe -CH₂-pyrazolyle, -CH₂-pyridinyle, -CH₂-thiényle ou -CH₂-isoxazolyle qui est facultativement substitué avec un ou plusieurs groupes halogéno, cyano, halogénalkoxy en C₁ à C₆, halogénalkyle en C₁ à C₆, alkyle en C₁ à C₆ ou alkoxy en C₁ à C₆ ;
- C (R^{a}R^{b}) -CONH-(cycloalkyle en C₃ à C₁₀) ; ou
- tétrahydropyrannyle facultativement substitué avec un
ou plusieurs groupes alkyle en C₁ à C₆ ;
R^{a} et R^{b} représentent indépendamment un atome d'hydrogène ou un groupe méthyle ou bien, conjointement avec l'atome de carbone auquel ils sont fixés forment un groupe cyclopropyle ou cyclohexyle ;
ou un de ses sels ou produits de solvatation pharmaceutiquement acceptables.

2. Composé suivant la revendication 1, dans lequel R⁴ représente un groupe -alkyle en C₁ à C₁₀ facultativement substitué avec un ou plusieurs groupes halogéno, alkoxy en C₁ à C₆, halogénalkoxy en C₁ à C₆ ou -S-alkyle en C₁ à C₆.

3. Composé suivant la revendication 1, qui est :
le 2,2-dioxyde de 6-éthyl-*N*-[(1*S*, 2*R*)-2-hydroxy-3-({[3-(méthyloxy)phényl]méthyl}amino-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-(tétrahydro-2*H*-pyranne-4-ylamino)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-({[1-(2,2,2-trifluoréthyl)-1*H*-pyrazole-4-yl]méthyl}-amino)propyl]-1-méthyl-1,3,4,6-tétrahydro[1,2]thiazépino-[5,4,3-*cd*]indole-8-carboxamide ;
le 2,2-dioxyde de 6-éthyl-*N*-{(1*S*,2*R*)-2-hydroxy-1-(phényl-méthyl)-3-[(phénylméthyl)amino]propyl}-1-méthyl-1,3,4,6-tétrahydro[1,2]thiazépino[5,4,3-*cd*]indole-8-carboxamide ;
le 2,2-dioxyde de 6-éthyl-*N*-{(1*S*,2*R*)-2-hydroxy-1-(phényl-méthyl)-3-[(4-pyridinylméthyl)amino]propyl}-1-méthyl-1,3,4,6-tétrahydro [1, 2]thiazépino[5,4,3-*cd*]indole-8-carboxamide ;
le 2,2-dioxyde de 6-éthyl-*N*-{(1*S*,2*R*)-2-hydroxy-1-(phényl-méthyl)-3-[(3-pyridinylméthyl)amino]propyl}-1-méthyl-1,3,4,6-tétrahydro[1,2]thiazépino[5,4,3-*cd*]indole-8-carboxamide ;
le 2,2-dioxyde de *N*-[(1*S*,2*R*)-3-[(2,2-diméthyltétrahydro-2H-pyranne-4-yl)amino]-2-hydroxy-1-(phénylméthyl)propyl]-6-éthyl-1-méthyl-1,3,4-6-tétrahydro[1,2]thiazépino[5,4,3-*cd*]-indole-8-carboxamide ;
le 2,2-dioxyde de 6-éthyl-*N*-[(1*S*,2*R*)-3-{[(3-éthyl-5-isoxazolyl)métyl]amino}-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de *N*-[(1*S*,2*R*)-3-(cyclobutylamino)-2-hydroxy-1-(phénylméthyl)propyl]-6-éthyl-1-méthyl-1,3,4,6-tétrahydro[1,2]thiazépino[5,4,3-*cd*]indole-8-carboxamide;
le 2,2-dioxyde de *N*-[(1*S*,2*R*)-3-[(4,4-difluorocyclohexyl)-amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-[(2-fluoréthyl)-amino]-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-[(2,2,3,3,3-pentafluoropropyl)amino]-1-(phénylméthyl)propyl]-1-méthyl-1,3,4,6-tétrahydro[1,2]thiazépino[5,4,3-cd]indole-8-carboxamide ;
le 2,2-dioxyde de 6-éthyl-*N*-[(1*S*,2*R*)-3-{[(5-éthyl-3-thiényl)méthyl]amino}-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[2-(méthyloxy)éthyl]amino}-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-{(1*S*,2*R*)-2-hydroxy-1-(phényl-méthyl)-3-[(2,2,2-trifluoréthyl)amino]propyl}-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-(éthylamino)-2-hydroxy-1-(phénylméthyl)propyl]-1-méthyl-1,3,4,6-tétrahydro-[1, 2] thiazépino [5, 4, 3-*cd*] indole-8-carboxamide ;
le 2,2-dioxyde de *N*-[(1*S*,2*R*)-3-[(cyclopropylméthyl)-amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de *N*-[(1*S*,2*R*)-3-(cyclohexylamino)-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de *N*-[(1*S*,2*R*)-3-(3-cyclopentène-1-yl-amino)-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-{[2-(éthylthio)-éthyl]amino}-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-[(4-méthylcyclohexyl)amino]-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-({[3-(trifluorométhyl)phényl]méthyl}amino)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-[(1-propylbutyl)amino]propyl}-1-méthyl-1,3,4,6-tétrahydro[1,2]thiazépino[5,4,3-*cd*]indole-8-carboxamide ;
le 2,2-dioxyde de *N*-[(1*S*,2*R*)-3-[(4,4-diméthylcyclohexyl)-amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-(2-propyne-1-ylamino)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-(2-propén-1-ylamino)propyl]-1-méthyl-1,3,4,6-tétrahydro[1,2]thiazépino[5,4,3-*cd*]indole-8-carboxamide ;
le 2,2-dioxyde de *N*-[(1*S*,2*R*)-3-[(3,3-diméthylbutyl)-amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-[(3,3,5,5-tétraméthylcyclohexyl)amino]propyl}-1-méthyl-1,3,4,6-tétrahydro[1,2]thiazépino[5,4,3-*cd*]indole-8-carboxamide ;
le 2,2-dioxyde de *N*-[(1*S*,2*R*)-3-[(1,5-diméthylhexyl)-amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-(propylamino)propyl]-1-méthyl-1,3,4,6-tétrahydro-[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-[(3,3,3-trifluoropropyl)amino]propyl]-1-méthyl-1,3,4,6-tétrahydro[1,2]thiazépino[5,4,3-cd]indole-8-carboxamide ;
le 2,2-dioxyde de *N*-[(1*S*,2*R*)-3-[(2,2-difluoréthyl)amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-[(2-éthylbutyl)-amino]-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-[(3-méthylbutyl)amino]-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-[(2,2,6,6-tétraméthylcyclohexyl)amino]propyl}-1 - méthyl-1,3,4,6-tétrahydro[1,2]thiazépino[5,4,3-*cd*]indole-8-carboxamide ;
le 2,2-dioxyde de *N*-[(1*S*,2*R*)-3-[(2,2-diméthylcyclohexyl)-amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[2-(méthylthio)éthyl]amino}-1-(phénylméthyl)propyl]-1-méthyl-1,3,4,6-tétrahydro[1,2]thiazépino[5,4,3-cd]indole-8-carboxamide ;
le 2,2-dioxyde de *N*-[(1*S*,2*R*)-3-[(2-cyclohexyléthyl)amino]-2-hydroxy-1-(phénylméthyl)propyl]-6-éthyl-1-méthyl-1,3,4,6-tétrahydro[1,2]thiazépino[5,4,3-cd]indole-8-carboxamide ;
le 2,2-dioxyde de 6-éthyl-*N*-[(1*S*,2*R*)-2-hydroxy-3-[(2-méthyl-2-propén-1-yl)amino]-1-(phénylméthyl)propyl]-1-méthyl-1,3,4,6-tétrahydro[1,2]thiazépino[5,4,3-cd]indole-8-carboxamide ;
le 2,2-dioxyde de *N*-[(1*S*,2*R*)-3-(3-butén-1-ylamino)-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de *N*-[(1*S*,2*R*)-3-(cycloheptylamino)-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-((1*S*,2*R*)-2-hydroxy-1-(phényl-méthyl)-3-{[2-(propyloxy)éthyl]amino}propyl)-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-[(1-éthynyl-cyclohexyl)amino]-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-[(4-méthylphényl)amino]-1-(phénylméthyl)propyl]-1-méthyl-1,3,4,6-tétrahydro [1,2] thiazépino [5,4,3-*cd*] indole-8-carboxamide ;
le 2,2-dioxyde de 6-éthyl-*N*-[(1*S*,2*R*)-2-hydroxy-3-[(1-méthylcyclohexyl)amino]-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-[(1-éthyl-cyclohexyl)amino]-2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-[(1-propylcyclohexyl)amino]propyl}-1-méthyl-1,3,4,6-tétrahydro[1,2]thiazépino[5,4,3-*cd*]indole-8-carboxamide ;
le 2,2-dioxyde de *N*-[(1*S*,2*R*)-3-({2-[(1,1-diméthyléthyl)-thio]éthyl}amino)-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-1-(phényl-méthyl)-3-({2-[(2,2,2-trifluoréthyl)oxy]éthyl}amino)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-(phényl-amino)-1-(phénylméthyl)propyl]-1-méthyl-1,3,4,6-tétrahydro-[1,2]thiazépino[5,4,3-*cd*]indole-8-carboxamide;
le 2,2-dioxyde de 6-éthyl-*N*-[(1*S*,2*R*)-2-hydroxy-3-[(3-méthylphényl)amino]-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-[(2-méthylphényl)amino]-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-{[(1-éthyl-1*H-*pyrazole-4-yl)méthyl]amino}2-hydroxy-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-[(3-méthyl-2-butén-1-yl)amino]-1-(phénylméthyl)propyl]-1-méthyl-1,3,4,6-tétrahydro[1,2]thiazépino[5,4,3-*cd*]indole-8-carboxamide ;
le 2,2-dioxyde de *N*-[(1*S*,2*R*)-3-[(2-chlorophényl)amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[2-(méthyloxy)phényl]amino}-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[4-(méthyloxy)phényl]amino}-1-(phénylméthyl)propyl]-1-méthyl-1,3,4,6-tétrahydro[1,2]thiazépino[5,4,3-*cd*]indole-8-carboxamide ;
le 2,2-dioxyde de*N*-[(1*S*,2*R*)-3-[(3-chlorophényl)amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[(6-méthyl-2-pyridinyl)méthyl]amino}-1-(phénylméthyl)propyl]-1-méthyl-1, 3, 4, 6-tétrahydro [1, 2] thiazépino [5,4,3-*cd*] indole-8-carboxamide ;
le 2,2-dioxyde de *N*-[(1*S*,2*R*)-3-{[(1*S*)-2-(cyclohexyl-amino)-1-méthyl-2-oxoéthyl]amino}-2-hydroxy-1-(phénylméthyl)-propyl]-6-éthyl-1-méthyl-1,3,4,6-tétrahydro[1,2]thiazépino-[5,4,3-*cd*]indole-8-carboxamide ;
le 2,2-dioxyde de 6-éthyl-*N*-[(1*S*,2*R*)-2-hydroxy-3-{[(1*R*)-1-méthylpropyl]amino}-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[(1*S*)-1-méthylpropyl]amino}-1-(phénylméthyl)propyl]-1-méthyl-1,3,4,6-tétrahydro [1,2] thiazépino [5,4,3-*cd*] indole-8-carboxamide ;
le 2,2-dioxyde de 6-éthyl-*N*-(1*S*,2*R*)-2-hydroxy-1-(phényl-méthyl)-3-[(2-pyridinylméthyl)amino]propyl}-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-2-hydroxy-3-{[2-méthyl-4-(méthyloxy)phényl]amino}-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-[(1-éthylcyclopropyl)-amino]-2-hydroxy-1-(phénylméthyl)propyl]-1-méthyl-1,3,4,6-tétrahydro [1,2] thiazépino [5,4,3-*cd*] indole-8-carboxamide ;
le 2,2-dioxyde de 6-éthyl-*N*-[(1*S*,2*R*)-2-hydroxy-3-(2-pentyne-1-ylamino)-1-(phénylméthyl)propyl]-1-méthyl-1,3,4,6-tétrahydro[1,2]thiazépino[5,4,3-*cd*]indole-8-carboxamide ;
le 2,2-dioxyde de 6-éthyl-*N*-[(1*S*,2*R*)-3-[(3-fluoropropyl)-amino]-2-hydroxy-1-(phénylméthyl)propyl]-1-méthyl-1,3,4,6-tétrahydro [1, 2] thiazépino [5, 4, 3-*cd*] indole-8-carboxamide ;
le 2,2-dioxyde de 6-éthyl-*N*-[(1*S*,2*R*)-2-hydroxy-3-[(1-méthylcyclopropyl)amino]-1-(phénylméthyl)propyl]-1-méthyl-1,3,4,6-tétrahydro[1,2]thiazépino[5,4,3-*cd*]indole-8-carboxamide;
le 2,2-dioxyde de *N*-[(1*S*,2*R*)-3-[(1,1-diméthyl-2-propyne-1-yl)amino]-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de *N*-[(1*S*,2*R*)-3-(cyclooctylamino)-2-hydroxy-1-(phénylméthyl)propyl]-6-éthyl-1-méthyl-1,3,4,6-tétrahydro[1,2]thiazépino[5,4,3-*cd*]indole-8-carboxamide ;
le 2,2-dioxyde de 1,6-diéthyl-*N*-[(1*S*,2*R*)-2-hydroxy-1-(phénylméthyl)-3-(tétrahydro-2*H*-pyranne-4-ylamino)propyl]-1,3,4,6-tétrahydro[1,2]thiazépino[5,4,3-*cd*]indole-8-carboxamide ;
ou un de ses sels ou produits de solvatation pharmaceutiquement acceptables.

4. Composition pharmaceutique comprenant un composé de formule (I) tel que défini dans l'une quelconque des revendications précédentes ou un de ses sels ou produits de solvatation pharmaceutiquement acceptables en mélange avec un ou plusieurs diluants ou supports pharmaceutiquement acceptables.

5. Composé de formule (I) suivant l'une quelconque des revendications 1 à 3 ou un de ses sels ou produits de solvatation pharmaceutiquement acceptables, destiné à être utilisé comme agent pharmaceutique.

6. Utilisation d'un composé de formule (I) tel que défini dans l'une quelconque des revendications 1 à 3 ou d'un de ses sels ou produits de solvatation pharmaceutiquement acceptables dans la production d'un médicament destiné au traitement de maladies **caractérisées par** des taux élevés de β-amyloïde ou de dépôts de β-amyloïde.
